# EUROPEAN PATENT SPECIFICATION

(11) **EP 4 436 483 B1**
(45) Date of publication and mention of the grant of the patent: **26.08.2026**
(21) Application number: 21965014.0
(22) Date of filing: 23.11.2021
(51) Int. Cl.: A61B 6/03, G01G 19/44, A61N 5/10

(54) **METHODS AND APPARATUS FOR RADIOTHERAPY PATIENT POSITIONING SUPPORT**
VERFAHREN UND VORRICHTUNG ZUR UNTERSTÜTZUNG DER POSITIONIERUNG EINES PATIENTEN IN DER RADIOTHERAPIE
PROCÉDÉS ET APPAREIL DE SUPPORT DE POSITIONNEMENT DE PATIENT POUR RADIOTHÉRAPIE

(43) Date of publication of application: 02.10.2024
(73) Proprietor: Elekta Beijing Medical Systems Co., Ltd., Beijing 102200 (CN)
(72) Inventor: LIU, Changhong, Beijing 102200 (CN); FU, Junlin, Beijing 102200 (CN); YU, Fei, Beijing 102200 (CN); GUO, Guangrui, Beijing 102200 (CN); CHEN, Jia, Beijing 102200 (CN); ZHENG, Xin, Beijing 102200 (CN)
(74) Representative: Thomas, Tomos Daniel
(86) International application number: PCT/CN2021/132398
(87) International publication number: WO 2023/092271

(56) References cited:
- WO-A1-2021/219830
- CN-A- 101 548 888
- CN-A- 107 536 620
- CN-A- 108 065 943
- CN-A- 109 806 508
- CN-A- 110 970 101
- CN-A- 113 545 794
- JP-A- 2013 242 149
- US-A1- 2009 003 522
- US-A1- 2018 289 574
- US-A1- 2020 061 392

## Description

The present disclosure relates generally to a radiotherapy apparatus, and in particular to a patient support for a radiotherapy apparatus for positioning a patient during the delivery or application of radiotherapy. Systems, apparatus, and methods for the characterisation and/or control of the patient support are disclosed herein.

### Background

A patient positioning apparatus may be used to position a patient in a scanning or treatment volume of a medical device. For example, in the field of radiotherapy, a patient positioning apparatus may be used to ensure the patient is correctly positioned with respect to a source of therapeutic radiation in accordance with a treatment plan. The patient positioning apparatus may be configured to move the patient in multiple of degrees of freedom, and this is particularly important in the field of radiotherapy to ensure optimal positioning of the patient and thus to ensure the prescribed dose of radiation is delivered accurately and optimally to a target region.

Modern radiotherapy treatment employs techniques to reduce the radiation dose to healthy tissue and thereby provide a safe treatment. For example, one approach to minimising a radiation dose received by healthy tissue surrounding a target region is to direct the radiation towards the target region from a plurality of different angles, for example by rotating a source of radiation around the subject using a rotating gantry. Radiation is emitted in a radiation plane which is co-incident with the plane of the gantry, around which the radiation source rotates to deliver radiation to an isocentre at the centre of the gantry, irrespective of the angular position of the radiation head around the gantry. The angles at which radiation is applied are selected such that each beam of radiation passes through the target region. In this way, a cumulative radiation dose may be built up at the target region over the course of a treatment arc, in which the radiation source rotates through a predetermined angle. Treatments that employ rotation of the gantry in this manner are known as coplanar.

Since the radiation is applied from a plurality of different angles centred on the target region, the specific healthy tissue the radiation passes through varies with the rotation of the radiation head. As such, build up of a high cumulative radiation dose is reduced in the healthy tissue. In other words, each unit volume of the healthy tissue receives a reduced radiation dose relative to a unit volume of the target region.

However, following a 180 degree rotation of the radiation source, subsequent radiation beams may begin to pass through regions of healthy tissue of a patient previously irradiated, and this may increase the cumulative radiation dose delivered to these regions of healthy tissue. Moreover, there may be cases where the target region is located behind a healthy organ, and as such it may be difficult or not possible to avoid the healthy organ and deliver the desired radiation dose directly to the target region. It is therefore desirable to enable additional degrees of freedom to reduce the radiation dose delivered to each region of healthy tissue.

One approach to providing an additional degree of freedom so as to spread the radiation dose received by healthy tissue surrounding a target region is to rotate the patient in a plane perpendicular to the plane of radiation as well as rotating the radiation source. Effectively, the angle of radiation varies both in the plane of the gantry and in a plane perpendicular to the gantry about a vertical z-axis. Such an approach is known as non-coplanar.

By rotating the patient in a non-coplanar manner about the vertical z-axis, the radiation dose delivered to each unit volume of healthy tissue in the patient can be further reduced. However, such rotation about the vertical z-axis may result in the target region being shifted away from the isocentre. Further degrees of freedom may therefore be desirable to enable such a shift to be compensated, in addition to enabling further reduction of the radiation dose received by healthy tissues.

Design requirements for a patient positioning apparatus may also call for adjustment of not only a rotation angle of the patient supporting apparatus, but also adjustment of translatory degrees of freedom such as the height and linear position of the patient support apparatus.

However, existing systems that are capable of providing one or more further degrees of freedom are expensive and bulky, due to the complex mechanisms required to enable the additional degree(s) of freedom.

Furthermore, to ensure optimised treatment, it is important to ensure that the patient position is adjusted accurately, i.e. that the patient's actual position is well aligned with an intended patient position. Several factors can interfere with this alignment between 'intended' and 'actual' position, for example mechanical flexing due to the patient's weight, and mechanical tolerances in each of the available degrees of freedom. As patient positioning systems become more complex, adjusting a patient's position according to one degree of freedom, for example a rotation, can have an effect on the accuracy of the patient's position in a translational degree of freedom, for example. Highly accurate control of the mechanisms that control patient positioning is essential in order to be most effective in reducing the exposure of healthy tissue to radiation. Such accuracy is difficult to achieve when introducing further degrees of freedom and mechanical complexity.

The present invention seeks to address these and other disadvantages encountered in the prior art.

WO2021219830A1 describes, in accordance with its abstract, a method of positioning a patient for radiotherapy treatment using a radiotherapy system. The method comprises determining a first target position for the patient for radiotherapy treatment; implementing a spatial relationship between the patient and at least a part of the radiotherapy device, at a first time, according to the first target position; providing radiotherapy treatment to the patient; determining a current position of the patient, at a second, subsequent time; and determining whether a change of a spatial relationship between the patient and at least a part of the radiotherapy device should be made, according to the first target position.

US20180289574A1 describes, in accordance with its abstract, a robotized bed which, may include a table for placing a patient, and a robot arm configured to support the table by a distal end, and including a base, a first movable element supported by the base and a second movable element coupled to the first movable element by a horizontally-rotating joint, wherein the robot arm is configured to move the table to a plurality of predetermined positions including a treatment position for a doctor to treat a patient, wherein the robotized bed is configured so that the robotic arm do not come in contact with the table, when the robot arm rotates the table while maintaining the table parallel to the horizontal plane.

US2020061392A1 describes, in accordance with its abstract, systems, devices, and methods for imaging-based calibration of radiation treatment couch position compensations.

US20090003522A1 describes, in accordance with its abstract, a method for providing radiation therapy to target tissue in a patient that provides for adjustment of the vertical position of the patient couch to account for errors introduced by the weight supported by the couch at its desired positions for treatment. The method further contemplates determining the initial location of the center of the target tissue with respect to the immobilization frame supporting the patient, to eliminate errors introduced by collateral position sensing equipment.

### Summary

An invention is set out in the independent claims. Optional features of the invention are defined in the dependent claims.

Disclosed herein is a computer-implemented method of controlling a patient positioning apparatus for a radiotherapy system, the patient positioning apparatus comprising a tiltable patient support apparatus. The method comprises receiving a reference position of the patient support apparatus and determining a position adjustment signal for controlling at least one actuator to adjust the position of the patient positioning apparatus from a current position to the reference position based on a parameter dependent on a physical characteristic of a patient, wherein the position adjustment signal is further based on a discrepancy between an estimated position and the reference position, wherein the estimated position is estimated at least partially based on a reading from a sensor, wherein the parameter dependent on a physical characteristic of the patient is determined using signals received from the sensor.

### Figures

Specific embodiments are now described, by way of example only, with reference to the drawings, in which:
Figure 1 depicts a radiotherapy device or apparatus comprising a patient positioning apparatus;
Figures 2a-c depict a patient positioning apparatus;
Figures 3a-b depict a patient positioning apparatus at different tilt angles;
Figures 4a-b depict opposing side views of the patient positioning apparatus;
Figure 5 depicts a rotation mechanism;
Figures 6a-b depict a cross section through a motion converter and a drive member of the rotation mechanism depicted in figure 5;
Figures 7a-7b depict a patient positioning apparatus at different tilt angles;
Figures 8a-8b depict a cross section through a drive member of the rotation mechanism depicted in figures 7a and 7b;
Figure 9 depicts a patent positioning apparatus comprising a sensor arrangement;
Figure 10 depicts the pitch and roll axis of an exemplary patient support apparatus;
Figures 11a-11b depict a sensing arrangement;
Figure 12 depicts a sensing arrangement;
Figure 13 shows a perspective view of an arrangement of a patient positioning apparatus;
Figure 14 shows a top view of the patient positioning apparatus of figure 13;
Figure 15A shows a side view from a long side of the patient positioning apparatus of figure 13;
Figure 15B shows a side view from a short side of the patient positioning apparatus of figure 13;
Figure 16a depicts a perspective view of an arrangement of a patient positioning apparatus;
Figure 16b depicts a perspective view of the patient positioning apparatus of figure 16a with a plate removed;
Figure 16c depicts a side view from a short side of the patient positioning apparatus of figure 16a and figure 16b;
Figure 17a depicts an actuator and a motion converter which may both form part of a tilting module according to the present disclosure;
Figure 17b depicts the actuator of figure 17a with the movement converter removed so as to better depict the actuator;
Figure 18a depicts a characterisation of a position discrepancy caused by pitch movement;
Figure 18b depicts a characterisation of a position discrepancy caused by roll movement;
Figure 19a depicts a characterisation of a remaining position discrepancy after the position discrepancy caused by pitch movement has been compensated;
Figure 19b depicts a remaining position discrepancy after the position discrepancy caused by roll movement has been compensated;
Figure 20 depicts a characterisation of a position discrepancy caused by rigid deformation;
Figure 21 depicts a system for determining position discrepancy caused by rigid deformation;
Figure 22 depicts a position of an inclinometer on a patient support apparatus;
Figure 23a depicts a method for characterising and/or controlling the accuracy of a patient positioning apparatus;
Figure 23b depicts a method for characterising and/or controlling the accuracy of a patient positioning apparatus; and
Figure 24 depicts a method for controlling a patient positioning apparatus.

### Detailed Description

The present application relates to a method and system for controlling a patient positioning apparatus, in particular a patient positioning apparatus for a radiotherapy system. The method and system provide improved characterisation of the positioning accuracy of the patient positioning apparatus, and accurate control of the position of the patient support apparatus and, accordingly, the position of the patient support surface. In some implementations, a position discrepancy between an ideal (or intended) position and an achieved (or actual) position is estimated. Once the discrepancy has been estimated, it can be compensated for. In particular, the position discrepancy is estimated and/or compensated according to a parameter based on a parameter derived from a physical characteristic of the patient, for example the patient's measured or estimated weight.

In known techniques, the position of the patient support surface, or table, in the treatment room can be detected in a number of different ways, which includes detection from a camera mounted to the ceiling of the room. These stereoscopic cameras are usually expensive and need calibration and QA on a regular basis to ensure the position of the table is being reported correctly. In addition, there is the potential for something to be positioned such that the view of the ceiling mounted camera is obstructed, causing the system to be unaware of the position of the patient positioning system. Several advantages are achieved by the methods and/or systems of the present application. The approach disclosed herein beneficially allows a highly complex patient positioning system to be controlled with an accuracy that is dependent upon the measurement accuracy of a small number of motors and/or sensors, which are typically highly accurate. Furthermore, using the approach disclosed herein, the accuracy can be estimated and/or controlled according to individual patient characteristics, such as patient weight, or mass. Such an approach can greatly improve patient workflow compared to known techniques, which rely either on repositioning the patient on the support surface or on using optical detection or monitoring of the position.

The present application also relates to a patient positioning apparatus for a medical device, preferably a radiotherapy device. The patient positioning apparatus comprises a patient support apparatus configured to rotate about a support structure. In use, a patient lies on an upper surface of the patient support apparatus. The support structure extends between the patient support apparatus and the floor of the treatment room in order to provide support to the patient support apparatus, and in particular in order to support the patient support apparatus above the floor of the treatment room.

Rather than providing a rotation mechanism as part of the patient support apparatus, as in prior arrangements, the rotation mechanism of the present disclosure is instead attached to, and supported by, this support structure. By moving the rotation mechanism out from the same plane as the patient support apparatus and fixing the rotation mechanism to the support structure, e.g. to support legs which are configured to bear the weight of the patient support apparatus, the hop-on height of the patient positioning apparatus is reduced, and the degree to which the patient support apparatus can be tilted is increased as there are fewer mechanical impediments to the rotation. Also, by providing a rotation mechanism attached to, and supported by, the support structure in this way, the mechanism is easier to access and maintain.

In some implementations of this apparatus, the patient support apparatus comprises a support surface which is moveable in a linear direction with respect to a patient support base. By removing the rotation mechanism from between these two layers and instead positioning it underneath the patient support base in the manner described herein, this linear movement is greatly facilitated and a simpler mechanism may be provided. In particular, it is no longer required to translate the entire rotation mechanism, which can instead remain stationary as a patient support surface translates with respect to the patient support base.

Also, in prior designs, during rotation of the patient support apparatus the entire weight of the patient support apparatus must be borne by the rotation mechanism. In the present design, this weight may instead be borne by the support structure. Accordingly, mechanical wear and tear is reduced, which in turn reduces the chances of breakdown and increases the longevity of the apparatus.

### A radiotherapy device

The patient positioning apparatuses described herein may be used in conjunction with a medical device, for example an imaging device. In a preferred implementation, the medical device is a radiotherapy device.

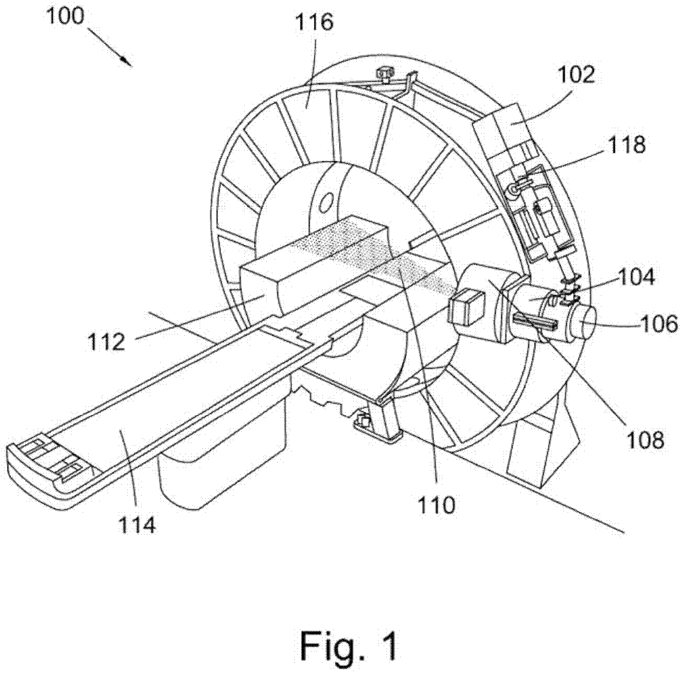

Figure 1 depicts a radiotherapy device suitable for delivering, and configured to deliver, a beam of radiation to a patient during radiotherapy treatment. The device and its constituent components will be described generally for the purpose of providing useful accompanying information for the present invention. The patient positioning apparatus of the present disclosure may be used to position a patient in the treatment and / or imaging volume of the device depicted in figure 1.

The device 100 depicted in figure 1 is a medical device. The medical device is a MR-linac. The device 100 comprises both MR imaging apparatus 112 and radiotherapy (RT) apparatus which may comprise a linac device. The MR imaging apparatus 112 is shown in cross-section in the diagram. In operation, the MR scanner produces MR images of the patient, and the linac device produces and shapes a beam of radiation and directs it toward a target region within a patient's body in accordance with a radiotherapy treatment plan. The depicted device does not have the usual 'housing' which would cover the MR imaging apparatus 112 and RT apparatus in a commercial setting such as a hospital.

The MR-linac device depicted in figure 1 comprises a source of radiofrequency waves 102, a waveguide 104, a source of electrons 106, a source of radiation 106, a collimator 108 such as a multi-leaf collimator configured to collimate and shape the beam, MR imaging apparatus 112, and a patient positioning apparatus comprising a patient support apparatus, the patient support apparatus comprising a patient support surface 114. In use, the device would also comprise a housing (not shown) which, together with the ring-shaped gantry, defines a bore. The moveable support surface 114 can be used to move a patient, or other subject, into the bore when an MR scan and/or when radiotherapy is to commence. The MR imaging apparatus 112, RT apparatus, and a patient support surface actuator are communicatively coupled to a controller or processor. The controller is also communicatively coupled to a memory device comprising computer-executable instructions which may be executed by the controller.

The RT apparatus comprises a source of radiation and a radiation detector (not shown). Typically, the radiation detector is positioned diametrically opposed to the radiation source. The radiation detector is suitable for, and configured to, produce radiation intensity data. In particular, the radiation detector is positioned and configured to detect the intensity of radiation which has passed through the subject. The radiation detector may also be described as radiation detecting means, and may form part of a portal imaging system.

The radiation source may comprise a beam generation system. For a linac, the beam generation system may comprise a source of RF energy 102, an electron gun 106, and a waveguide 104. The radiation source is attached to the rotatable gantry 116 so as to rotate with the gantry 116. In this way, the radiation source is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104.

Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

Once the electrons have been accelerated, they may pass into a flight tube. The flight tube may be connected to the waveguide by a connecting tube. This connecting tube or connecting structure may be called a drift tube. The electrons travel toward a heavy metal target which may comprise, for example, tungsten. Whilst the electrons travel through the flight tube, an arrangement of focusing magnets act to direct and focus the beam on the target.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

The source of radiation is configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multi-leaf collimator 108, before it passes into the patient as part of radiotherapy treatment.

In some implementations, the source of radiation is configured to emit either an X-ray beam or an electron particle beam. Such implementations allow the device to provide electron beam therapy, i.e. a type of external beam therapy where electrons, rather than X-rays, are directed toward the target region. It is possible to 'swap' between a first mode in which X-rays are emitted and a second mode in which electrons are emitted by adjusting the components of the linac. In essence, it is possible to swap between the first and second mode by moving the heavy metal target in or out of the electron beam path and replacing it with a so-called 'electron window'. The electron window is substantially transparent to electrons and allows electrons to exit the flight tube.

The subject or patient support surface 114 is configured to move between a first position substantially outside the bore, and a second position substantially inside the bore. In the first position, a patient or subject can mount the patient support surface. The support surface 114, and patient, can then be moved inside the bore, to the second position, in order for the patient to be imaged by the MR imaging apparatus 112 and/or imaged or treated using the RT apparatus. The movement of the patient support surface is effected and controlled by a patient support surface actuator, which may be described as an actuation mechanism. The actuation mechanism is configured to move the patient support surface in a direction parallel to, and defined by, the central axis of the bore. The terms subject and patient are used interchangeably herein such that the patient support surface can also be described as a patient support surface. The patient support surface may also be referred to as a moveable or adjustable couch or table.

The radiotherapy apparatus / device depicted in figure 1 also comprises MR imaging apparatus 112. The MR imaging apparatus 112 is configured to obtain images of a subject positioned, i.e. located, on the patient support surface 114. The MR imaging apparatus 112 may also be referred to as the MR imager. The MR imaging apparatus 112 may be a conventional MR imaging apparatus operating in a known manner to obtain MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the controller.

The controller is a computer, processor, or other processing apparatus. The controller may be formed by several discrete processors; for example, the controller may comprise an MR imaging apparatus processor, which controls the MR imaging apparatus 110; an RT apparatus processor, which controls the operation of the RT apparatus; and a patient support surface processor which controls the operation and actuation of the patient support surface. The controller is communicatively coupled to a memory, e.g. a computer readable medium.

The linac device also comprises several other components and systems as will be understood by the skilled person. For example, in order to ensure the linac does not leak radiation, appropriate shielding is also provided.

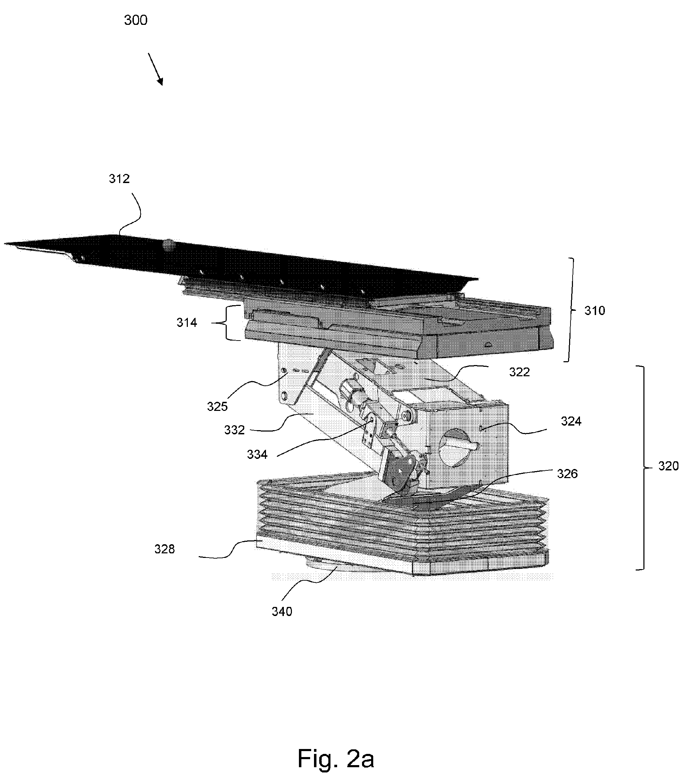

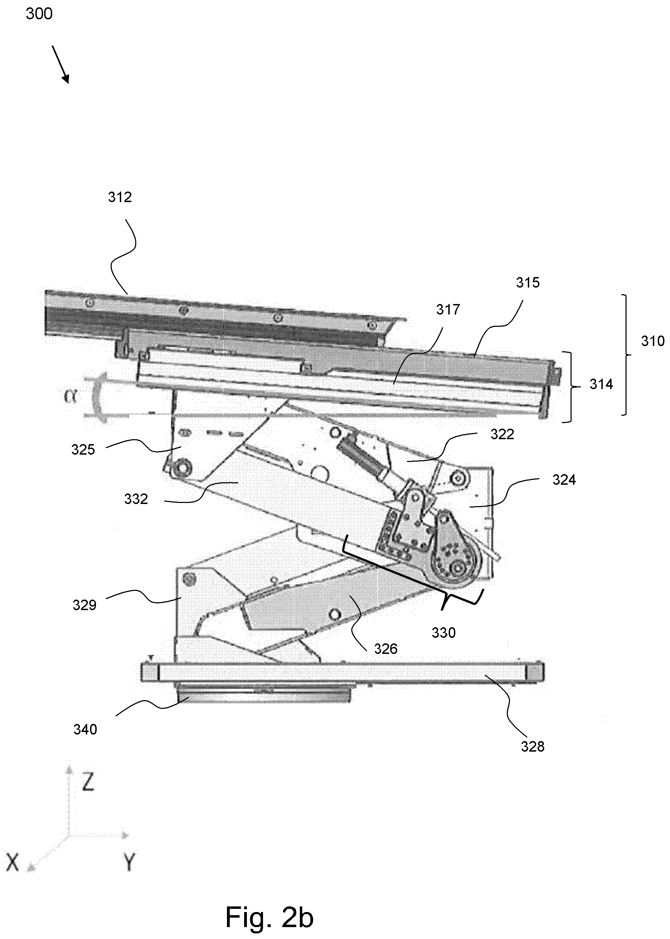

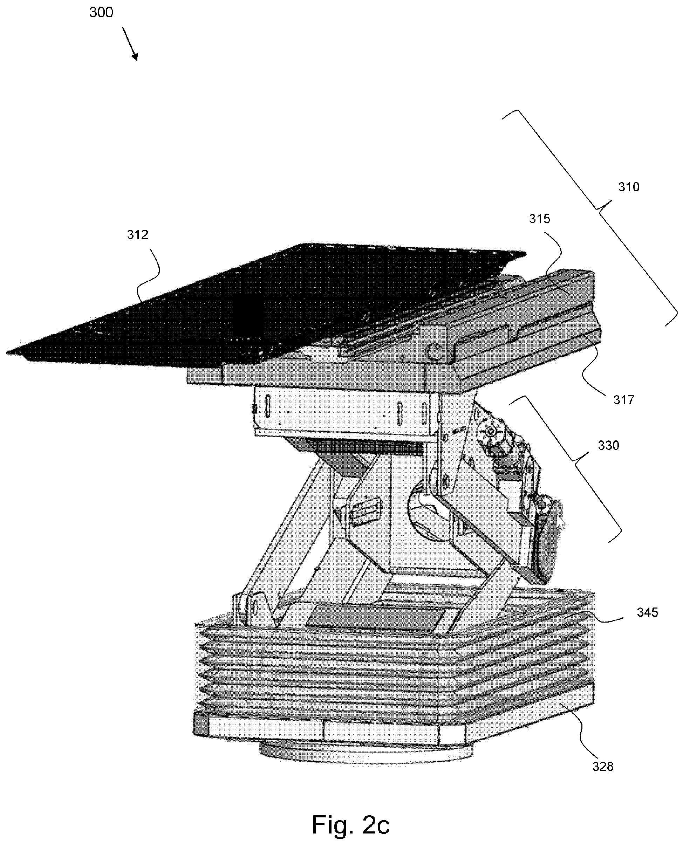

Figures 2a, 2b, and 2c depict a patient positioning apparatus 300 according to the present disclosure. Figure 2a depicts an angled rear view of the patient positioning apparatus 300. Figure 2b depicts a side-on view of the positioning apparatus 300. Figure 2c depicts an angled front view of the positioning apparatus 300. The patient positioning apparatus 300 comprises a patient support apparatus 310 and a support structure 320. The support structure 320 is configured to support the patient support apparatus 310 above a floor, such as the floor of a treatment room. The support structure 320 may be configured to provide this support, in part, by means of a base 328 which contacts the floor, and/or which is embeddable within the floor. In the implementation depicted in figures 2a-c, the support structure comprises a first, or upper, supporting leg 322, a support element 324, and a second, or lower, supporting leg 326.

The patient positioning apparatus 300 also comprises a rotation mechanism. The rotation mechanism is configured to tilt, i.e. rotate, the patient support apparatus 310. The rotation is made with respect to a horizontal plane, or equivalently with respect to the floor of the treatment room, in order to adjust a tilt angle, for example a pitch angle marked α in figure 2b. In the implementation depicted in figures 2a-c, the rotation mechanism comprises a drive member 332 and an actuation mechanism 330. The rotation mechanism is configured to impart a force, via the drive member 332, to an underside of the patient support apparatus 310 to thereby rotate the patient support apparatus 310 with respect to the support structure 320.

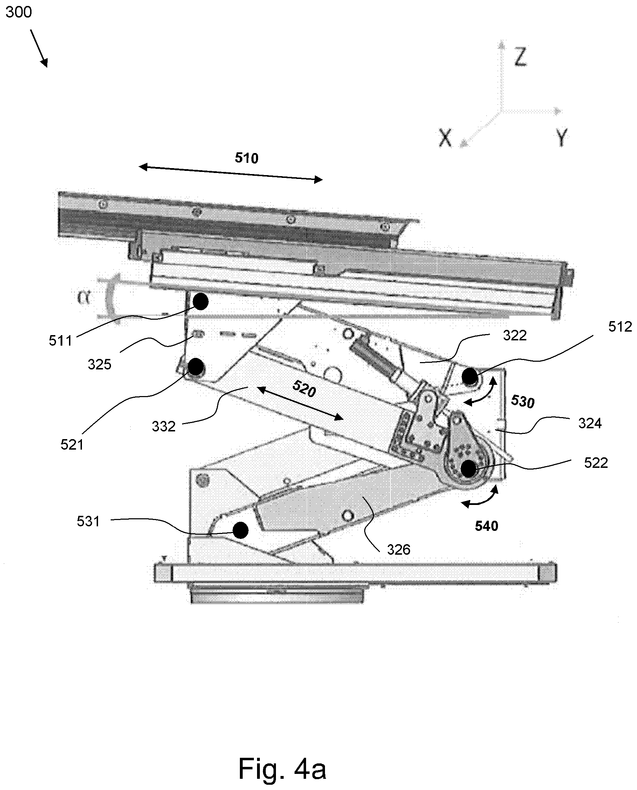

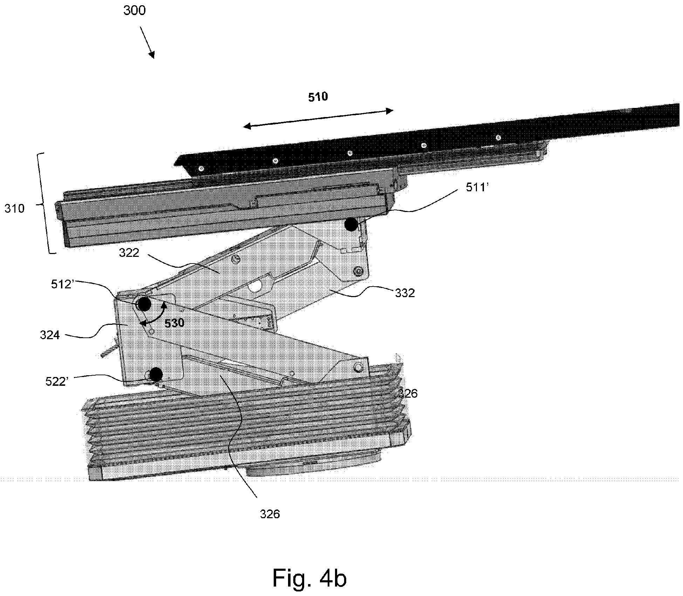

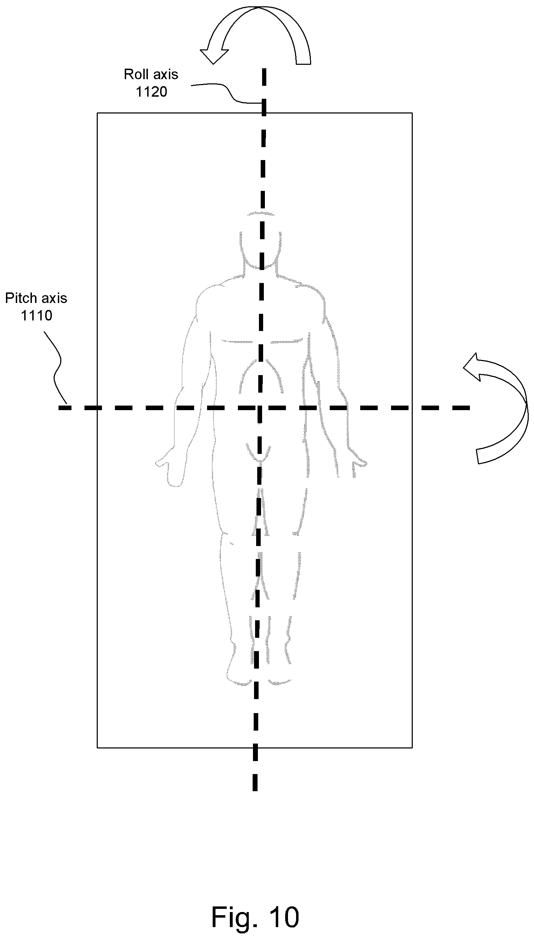

The patient support apparatus 310 is configured to support a patient. The patient support apparatus 310 comprises a patient support surface 312 and a patient support base 314. In use of the apparatus, a patient may lie on the patient support surface 312. In other words, in use, the patient contacts an upper surface of the patient support apparatus 310. The patient support surface 312 can be moved linearly with respect to the patient support base 314 along an axis parallel with the longitudinal axis of the patient support apparatus 310. The directions of this linear movement are indicated by the double-headed arrow 510 in figure 4a and figure 4b. The patient positioning apparatus 300 may be configured to rotate the patient support surface 312 with respect to one or both of a pitch and a roll rotation axis (see figure 10 and accompanying description below). In such an implementation, the axis of linear movement of the patient support surface 312 with respect to the patient support base 314 is parallel with and/or aligns with the roll rotation axis.

This movement may be controlled via a linear actuator or suitable actuation mechanism 330, and the patient support surface 312 is coupled with other components of the patient support apparatus 310, such as the patient support base 314, in order to facilitate this movement. This linear movement may be described as a translation. In other words, the patient support surface 312 is configured to translate linearly with respect to the patient support base 314.

For example, as can be seen best in figure 2c, the patient support base 314 may comprise upper base structure 315, such as an upper lateral sledge, and lower base structure 317, such as a lower lateral sledge. Together, the upper and lower base structures 315 and 317 form the patient support base 314. The patient support surface 312 comprises a longitudinal extension, or ridge. The longitudinal extension extends downward from a lower surface of the patient support surface. The patient support base 314, and in particular the upper base structure 315, comprises a corresponding longitudinal groove. The longitudinal groove is formed on an upper surface of the upper base structure 315. The longitudinal ridge is inserted in the groove in order to couple the patient support surface 312 to the upper base structure 315, but so as to allow movement of the patient support surface 312 with respect to the patient support base 324 along an axis defined by the longitudinal groove. Movement of the patient support surface 312 with respect to the patient support base is controlled via a linear actuator, such that the patient support surface 312 is configured to move in a longitudinal, linear manner with respect to the patient support base 314 and the other components of the patient positioning apparatus 300. It will be appreciated that the ridge and groove arrangement may be swapped in some implementations such that the support surface comprises the groove and the support base comprises the corresponding ridge.

In addition or as an alternative to a longitudinal movement, the patient positioning surface may also be configured to move laterally. This movement is perpendicular to the longitudinal movement, and can be controlled via movement of the upper lateral sledge 315 with respect to the lower lateral sledge 317. This movement can be effected by actuators in a known way. In summary then, the patient support surface 312 may be configured to move in any, all, or a combination of three translator degrees of freedom: height, a longitudinal movement (parallel to roll axis 1120 shown in figure 10) and a lateral movement (parallel to pitch axis 1110 shown in figure 10).

The rotation mechanism is configured to rotate the patient support apparatus 310, i.e. both the patient support surface 312 and the patient support base 314. Because the rotation mechanism is coupled to and supported by the support structure 320 and positioned underneath the patient support apparatus 310, rather than forming part of the patient support apparatus 310 as in previous designs, the weight and size of the patient support apparatus 310 can be significantly reduced. Because the rotation mechanism does not form part of the patient support apparatus 310, it is not necessary to translate the entire patient support apparatus 310 (including the rotation mechanism) as part of the linear translation of the patient support surface 312, as in previous designs. The rotational coupling which defines the rotation axis of the patient support apparatus 310 is positioned underneath the main body of the patient support apparatus 310, and in particular the base of the patient support apparatus 310 is coupled to a support structure 320 configure. Thereby, the linear translation of the patient can be achieved by translating a relatively light patient support surface 312 with respect to the patient support base 314. Accordingly, the load which the translation motor must bear is reduced, while the ability to control the tilt angle of the patient support apparatus 310 is maintained.

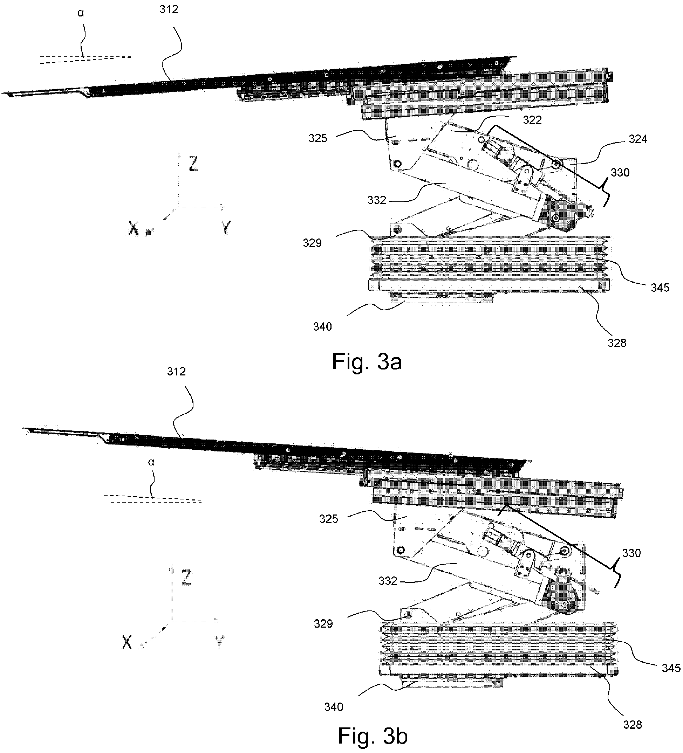

The support structure 320 is configured to bear the weight of the patient support apparatus 310, as well as a patient positioned on the patient support surface 312. Multiple implementations of the support structure 320 are envisaged. In the implementation depicted in figures 2 and 3, the support structure 320 comprises an upper element coupled to an underside of the patient support apparatus 310, and a lower element coupled to the base. The upper element may be a first supporting leg 322 and the lower element may be a second supporting leg 326 coupled to the base. The first supporting leg 322 and second supporting leg 326 are coupled to one another via a support element 324. The support element 324 may be referred to as a support block or anchor element herein.

The patient support apparatus 310 is rotationally coupled to the support structure 320 to allow rotation about a rotation axis. In a simple implementation, the support structure 320 may be coupled to the patient support apparatus 310 via the interaction between a shaft and one or more bearings which receive the shaft. For example, the one or more bearings may be mounted to an underside of the patient support apparatus 310, and configured to receive a shaft which forms part of the support surface. For example, an upper region of the first supporting leg 322 may culminate in a double-ended shaft, with each end of the shaft being received in a bearing mounted to a base of the patient support apparatus 310. In this implementation, the orientation of the shaft and bearings defines an axis of rotation about which the patient support apparatus 310 may rotate with respect to the support structure 320. Other implementations include a ball-joint, or any other mechanical connection that allows rotation of the patient support apparatus 310 with respect to the support surface via a rotation axis.

A second, or lower, supporting leg is coupled to the base. The second supporting leg 326 may be fixedly attached to the base. Alternatively, the coupling may be achieved via a lower coupling element and the second supporting leg 326 may be configured to rotate with respect to the lower coupling element as part of a height adjustment mechanism. The lower coupling element extends upward out of the plane of the base, allowing the second supporting leg 326 to be coupled to the lower coupling element to define a rotation axis parallel with the rotation axis about which the patient support apparatus 310 rotates with respect to the first supporting leg 322.

The support structure 320 may also comprise a height adjustment mechanism (not shown in the figures). The height adjustment mechanism is configured to adjust the height, i.e. vertical distance, of the patient support apparatus 310 above the floor or base. The height adjustment mechanism comprises one or more motor mechanisms. An upper motor mechanism may be positioned within, form part of, or be coupled to, the support element 324. A lower motor mechanism may be positioned within, form part of, or be coupled to, the lower coupling element.

The height adjustment mechanism may be formed by one or multiple different mechanisms. In the implementation depicted in figures 2a-c and 3a-b, the height adjustment mechanism is configured to adjust the vertical distance between the support element 324 and the patient support apparatus 310 by actuating the first supporting leg 322. Thereby, the height of the patient support apparatus 310 above the floor is increased. The height adjustment mechanism comprises a rotational mechanism or motor configured to produce a rotary motion of the first supporting leg 322 with respect to the support element 324. This may be a rotary hydraulic motor. This rotary motor is housed within the support element 324. It will be appreciated that by rotating the first supporting leg 322 clockwise from the perspective shown in figure 3, the height of the patient support apparatus 310 above the floor / base is increased.

Optionally, an additional rotary motor may be provided. This rotary motor may be referred to as a 'lower' rotary motor in contrast to the 'upper' rotary motor described above. The lower rotary motor is also housed within the support element 324 and is configured to drive rotation with respect to the support element 324 and the lower leg 326. The height adjustment mechanism may thereby also be configured to adjust the vertical distance between the support element 324 and the base 328 and/or floor of the treatment room, by actuating the second supporting leg 326 using this lower rotary motor. Thereby, the height of the patient support apparatus 310 is adjusted. By synchronously driving rotation using both the upper and the lower rotary motor, the vertical height of the patient support surface 312 may be adjusted.

For example, the height adjustment mechanism may comprise a lower rotational mechanism or motor, e.g. a rotary hydraulic motor, configured to produce a rotary motion of the second supporting leg 326 with respect to the support structure 324. It will be appreciated that by rotating the second supporting leg 326 anti-clockwise, from the perspective shown in figure 2b, the height of the patient support apparatus 310 above the base is increased.

The height adjustment mechanism is configured to control a height of the patient support apparatus 310 above the floor of the treatment room. As described above, the patient support apparatus 310, and in particular the base of the patient support apparatus 310, is rotationally coupled to the support structure 320 to allow rotation about a rotation axis. By adjusting the height of the patient support apparatus 310 above the floor of the treatment room using the height adjustment mechanism, the height of this rotation axis can also be adjusted.

While a support structure 320 and height adjustment mechanism has been described which comprises a mechanism capable of rotating one or a plurality of supporting legs about rotation axes in order to adjust the height of the patient support apparatus 310, the height adjustment mechanism may take multiple forms. For example, the height adjustment mechanism may comprise an arrangement of hydraulic pistons positioned and configured to adjust the height of the patient support apparatus 310. An alternative implementation may involve a scissor lift mechanism. The skilled person will be aware of other possible ways in which the height of a patient support apparatus 310 may be adjusted. Regardless of the specific implementation of the support structure 320 and/or height adjustment mechanism, the rotation mechanism is coupled to the support structure 320 and is configured to impart a force to an underside of the patient support apparatus 310 in order to rotate the patient support apparatus 310 with respect to the support structure 320.

In some implementations, the positioning apparatus 300 also comprises a skirt 345 (not shown in figure 2b) configured to cover the support structure 320 and rotation mechanism. The skirt 345 is connectable between the base 328 and the patient support apparatus 310. The skirt 345 has a flexibility, and in particular may have a concertina configured, i.e. be configured to extend, compress, or collapse in folds like those of a concertina. Thus, patients and clinicians are protected from injury by virtue of the moving mechanisms described herein. It is simpler to provide this protection using a simple skirt 345 by virtue of the present design, and in particular by virtue of the rotation mechanism being attached to and supported by the support structure. In figures 2a and 2c, the skirt is folded or compressed down away from the patient support apparatus 310 so that the support structure 320 and rotation mechanism can be seen.

As shown in figure 2b, three axes are defined and labelled X, Y, and Z respectively. Each axis is perpendicular to the other such that a three-dimensional coordinate system is formed by the axes. Changing the height of the patient support apparatus 310, for example by using the height adjustment mechanism described herein, changes the position of the patient support surface 312 in a direction parallel to the Z axis. Similarly, changing the longitudinal position of the patient support surface 312 corresponds to a change in position in a direction parallel to the Y axis. Changing the lateral position of the patient support surface 312 corresponds to a change in position in a direction parallel to the X axis. The patient support surface is accordingly translatable through three dimensions defined by the X, Y, and Z axes by using the mechanisms described herein.

The X, Y, and Z axes of figure 2b are also shown in other figures of the present disclosure, and may be referred to as the x-axis, y-axis, or z-axis. As will be appreciated, each of the various rotation and translation movements of the apparatus and systems disclosed herein can be described in terms of coordinates on these X, Y, and/or Z axes. Such a coordinate may be referred to as the patient support surface 312, or the patient support apparatus, having an "X position", "Y position" and/or "Z position". A change in any of the three coordinates may be referred to as changing or moving in the "X direction", "Y direction", and/or "Z direction".

### Rotation mechanism

The patient positioning apparatus 300 also comprises a rotation mechanism. The rotation mechanism is configured to rotate, i.e. tilt, the patient support apparatus 310. The rotation mechanism controls the angle of tilt. The rotation mechanism comprises a drive member 332 and an actuation mechanism 330. The rotation mechanism may also comprise a coupling element 325 which couples the drive member 332 to the patient support apparatus 310.

Two example extremes of tilt are depicted in figures 3a and 3b. The tilt angle is depicted using α. The tilt angle is measured with respect to a horizontal plane, or equivalently with respect to the floor of the treatment room. The tilt angle α is the angle which the patient support surface 312 makes with respect to the horizontal plane. In figures 3a and 3b, the 'type' of tilt is a rotation about a pitch axis, and may be referred to as a pitch rotation. In figure 3a, the front of the patient support surface 312 has been tilted down. The patient support apparatus of figure 3a is at maximum tilt angle α in this negative direction. In figure 3b, the front of the patient support surface 312 has been tilted upwards. The patient support apparatus of figure 3a is at maximum tilt angle α in this positive direction.

The rotation mechanism may take multiple different forms, though in each it is configured to impart a force to the patient support apparatus 310. By rotationally coupling the patient support apparatus 310 to the support structure 320 to define a rotation axis, and by providing a rotation mechanism which imparts a force to the patient support apparatus 310, a torque may be created about the rotation axis. By controlling the force imparted to the patient support apparatus 310, the torque created about the rotation axis may be controlled, and thus the degree of tilt of the patient support apparatus 310 about the rotation axis may be controlled. Herein, the rotation axis formed between the patient support apparatus 310 and the support structure 320 may be described as the principal rotation axis.

The movement of the drive member 332 occurs along the longitudinal axis of the drive member 332, and the drive member 332 can impart a 'push' force or a 'pull' force. The drive member 332 is coupled to an underside of the patient support apparatus 310, and the direction of tilt can thus be controlled by the direction of movement of the drive member 332. With reference to the viewpoint depicted in figures 2b and 3a, 3b, by pushing / moving the drive member 332 toward the underside of the patient support apparatus 310, a clockwise tilt about the principal axis can be achieved. By pulling / moving the drive member 332 away from the underside of the patient support apparatus 310, an anti-clockwise rotation about the principal axis may be achieved.

The drive member 332 is rotationally coupled to the coupling element 325 at a first (drive member 332) coupling point. The drive member 332 is rotationally coupled to the support element 324 at a second (drive member 332) coupling point. The actuation mechanism 330 is configured to move the drive member 332. More specifically, the actuation mechanism 330 is configured to control, e.g. adjust, a distance between the first and the second drive member coupling points. The first coupling point may be described as an upper coupling point, and the second coupling point may be described as a lower coupling point. The second coupling point is at a fixed position with respect to the support element 324, and therefore by adjusting this distance the location of the first coupling point is controlled by the actuation mechanism 330.

As the actuation mechanism 330 adjusts the distance between the first and second coupling point, the distance between the first coupling point and the support element 324 is also adjusted. By increasing the distance between the first and second coupling points, the first coupling point, and thus the coupling element, is pushed away from the support element 324 of the support structure 320. Conversely, by reducing the distance between the first and second coupling points, the first coupling point, and thus the coupling element 325, are pulled toward the support element 324 of the support structure 320.

The coupling element 325 extends along an underside of the patient support apparatus 310, in particular along an underside of the patient support base 314, in a direction of the longitudinal axis of the patient support apparatus 310. The coupling element 325 also extends away from an underside of the patient support apparatus 310 in a direction substantially perpendicular to the plane of the patient support apparatus 310. Extension in this direction provides sufficient surface area for the coupling element 325 to be coupled to the first supporting leg 332 at the first (drive member 332) coupling point. Optionally, the coupling member 325 may also be rotationally coupled to the support structure 330 to allow rotation of the coupling member 325 about the principal rotation axis.

### Summary of movement and the various axes of rotation

Above, the actions and configurations of a height adjustment mechanism and a rotation mechanism have been described. The resulting directions of movement and the driven axes of rotation may be summarised by reference to figures 4a and 4b. As shown in figure 4a, which depicts the same implementation shown in figures 2a-c and 3a-b, the drive member extends between the (upper) coupling element 325 and the support element 324, and is rotationally coupled to these elements. The drive member 332 is rotationally coupled to the coupling element 325 at a first (drive member) coupling point 521, and rotationally coupled to the support element 324 at a second (drive member) coupling point 522. The rotation mechanism is configured to adjust the distance between these two coupling points. Adjusting the distance between the first drive member coupling point 521 and the second drive member coupling point 522 causes the drive member 332 to move. Adjusting the distance between the first drive member 332 coupling point 521 and the second drive member coupling point 522 as measured along the longitudinal axis of the drive member 332 causes the drive member 532 to move along its longitudinal axis.

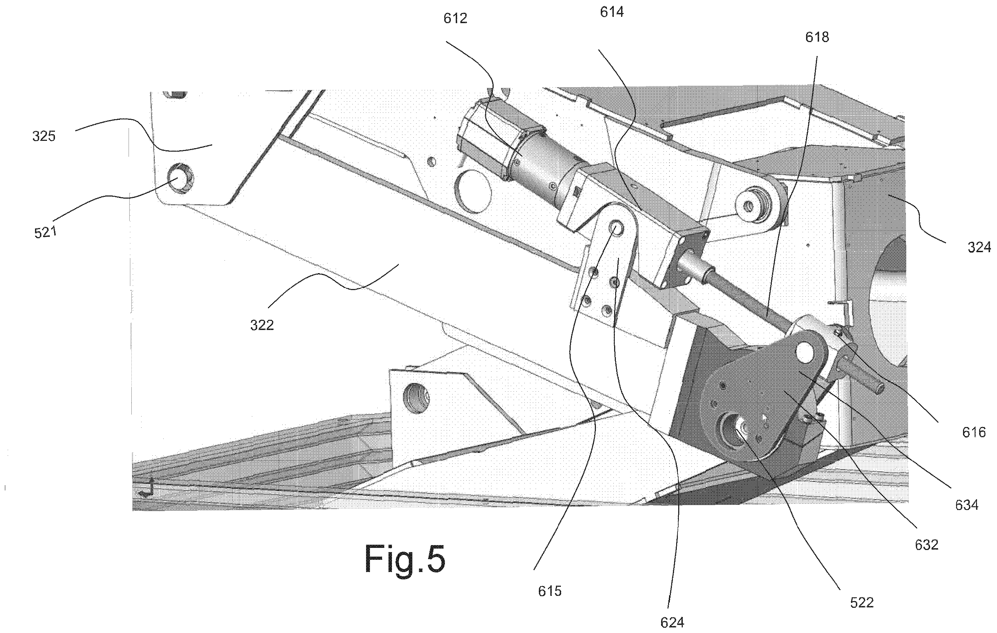

In an implementation, the drive member 332 comprises an aperture which extends in a direction along the longitudinal axis of the drive member 332, and the drive member 332 is rotationally coupled to the support element 324 by means of this aperture. The support element 324 comprises a fixed spindle, or axle, that defines the location of the second drive member coupling point 522. The fixed spindle, or axle, is fixedly attached to the support element 324 and its location is fixed with respect to the support element 324. The fixed spindle / axle slots into the aperture to allow rotation of the drive member 332 with respect to the supporting element 324. Thus, the second coupling point 522 is fixed with respect to the support element 324. However, by moving the drive member 332 in a direction along the longitudinal axis of the drive member 332, the position of the fixed spindle or axle within the aperture moves. The actuation mechanism 330 of the rotation mechanism controls this movement. Linear movement of the drive member 332 is depicted by arrow 520 in figure 4a. Specific implementations of the rotation mechanism and second (drive member) coupling point 522 are described herein with respect to figures 5, 6a-b, 7a-b, and 8a-b.

The upper supporting leg 322 extends between the underside of the patient support apparatus 310 and the support element 324. As with the drive member 332, the supporting leg 322 is rotationally coupled at each end. The upper supporting leg 322 is rotationally coupled to the base of the patient support apparatus 310at a first (supporting leg) coupling point 511. The first (supporting leg) coupling point 511 defines a principal, or primary, rotation axis. The first (supporting leg) coupling point 511 may be described as the principal, or primary, coupling point. Rotation about this principal axis adjusts the tilt angle α of the patient support apparatus. With respect to figure 10, the first (supporting leg) coupling point 511 may define the pitch rotation axis.

The upper supporting leg 322 is rotationally coupled to the support element 324 at a second (supporting leg) coupling point 512. The second (supporting leg) coupling point 512 is located above the second (drive member) coupling point 522 on the support element 324. The support element 324 acts as an anchor and may be held stationary (e.g. by the lower supporting leg 326) while the drive member 332 and the upper supporting leg 322 rotate.

By defining the first and second supporting leg coupling points 511, 512 and the first and second drive member coupling points 521, 522, it is possible to view these points as being the vertices of a parallelogram. The sides, or edges, of the parallelogram may be defined by first opposing sides (drive member 332 and supporting leg 322) and second opposing sides (coupling element 325 and support element 324). The rotation mechanism is configured to control the distance between the first and second drive member coupling points 521, 522 and thus is configured to adjust the length of one of the edges of the parallelogram. By adjusting this length, the angles made between the coupling member 325 and both the drive member 332 and the upper supporting leg 322 are adjusted. This adjusts the orientation of the coupling element 325 with respect to the supporting element 324. Because the coupling element 325 is fixedly attached to the base 314 of the patient support apparatus 310, this in turn causes the patient support apparatus 310 to rotate about the principal rotation axis.

The lower supporting leg 326 is rotationally coupled to the lower coupling element 329 at lower supporting leg coupling point 531. The lower supporting leg 326 is also rotationally coupled to the supporting element 324 at coupling point 522. Thus, the spindle or axle which defines coupling point 522 is configured to couple together the lower supporting leg 522, the supporting element 324, and the drive member 332.

Figure 4b is a side view depicting the opposite side of the patient positioning apparatus 300 in comparison to the side shown in figure 4a. This side is much like the side depicted in figure 4a, though the rotation mechanism is not positioned on this side. An axle, spindle or other rotational coupling member defines the position 511' at which the upper supporting leg 322 is rotationally coupled to the patient support apparatus 310. Like reference numerals depict like features.

The coupling points may be described as rotational coupling points, and may be defined by any of a number of rotational coupling mechanisms and structures, for example extending rods, axles or spindles which are received in a suitably sized, positioned and configured bearing or aperture. The specific manner in which each individual coupling point is achieved need not be described in detail as the skilled person will be familiar with ways in which to couple two elements together in order to achieve rotation.

While reference is made to coupling 'points', a limited and narrow meaning of the word 'point' is not intended. It should instead be understood that these coupling points define rotational axes. For example, the upper supporting leg 322 may be rotationally coupled to the support element 324 at two different points, e.g. the coupling point 512 depicted in figure 4a and the coupling point 512' depicted in figure 4b may be different points in space. However, these points 512, 512' together define a rotational axis about which the upper supporting leg 322 may rotate with respect to the supporting element 324.

As described above, the actuation mechanism 330 of the rotation mechanism is configured to impart a force to the patient support apparatus 310 via the drive member 332. This is accomplished by moving the drive member 332, which in turn is attached, i.e. coupled, to the underside of the patient support apparatus 310. The actuation mechanism 330 moves the drive member 332 in a direction defined by a longitudinal axis of the drive member 332. The drive force can be described as a push, or a pull, depending on its direction. The rotation mechanism is configured to push the drive member 332 toward the coupling element 325, as well as to pull the drive member 332 away from the coupling element 325. Movement of the drive member 332, as controlled by the actuation mechanism 330, is depicted using arrow 520 in figure 4a.

As described above, the height adjustment mechanism may control the height of the patient support apparatus 310 by controlling rotation of the upper supporting leg 322 with respect to the support element 324. This rotation occurs about the second supporting leg coupling point 512 and is driven by a rotary motor. This rotation is depicted by arrow 530 in figure 4. Alternatively, or additionally, the height adjustment mechanism may comprise another rotary motor configured to rotate the lower supporting leg 326 with respect to the support element 324. This rotation occurs around the second drive member coupling point 522 and is depicted by arrow 540 in figure 4. The height can thus be controlled by rotation of one or more rotary motors configured to rotate the supporting legs 322, 326 both upward and downward. By synchronously driving rotation about axes depicted by arrows 530 and 540, the vertical height of the patient support surface can be adjusted without also adjusting the longitudinal or lateral position of the patient support surface 312.

From the above description, it should be appreciated that the drive member 332 is configured to move in three degrees of freedom, a translatory degree of freedom along a longitudinal axis of the drive member 332 as described by arrow 520, and two rotational degrees of freedom. The drive member 322 may rotate about an axis defined by the first drive member coupling point 521 to define a first degree of freedom, and rotate about an axis defined by the second drive member coupling point 522 to define a second degree of freedom. The drive member 332 is driven in the translatory degree of freedom by the rotation mechanism 330. The drive member 332 is not driven about the rotational degrees of freedom, but instead freely rotates about these rotational axes as the height adjustment mechanism adjusts the height of the patient support apparatus 310. In more detail: as the height adjustment mechanism drives the upper supporting leg 322 upwards by rotating 530 the upper supporting leg 322 about its second coupling point 522, the height of the patient support apparatus increases. As an upper portion of the drive member 332 is coupled to an underside of the patient support apparatus via coupling element 325, the drive member is pulled upwards by movement of the patient support apparatus 310. To account for this movement, the drive member 332 rotates about its upper (first) coupling point 511 and its lower (second) coupling point 522. Thus, the drive member 332 is always positioned to effect tilting of the patient support apparatus 310 regardless of the height of the patient support apparatus 310.

Control of the height of the patient support apparatus 310 by the height adjustment mechanism is independent of the control of rotation of the patient support apparatus 310 by the rotation mechanism. Both movements are controllable by one or more processors.

The height adjustment mechanism is configured to control a height of the patient support apparatus 310 above the floor of the treatment room. It follows that the height adjustment mechanism is configured to control a height of the patient support apparatus 310 above the base 328 of the support structure 320. The height adjustment mechanism thereby also increases the height of the principal rotation axis (as defined by the principal coupling point / the first supporting leg coupling point 511). For example, with reference to figure 4a, it can be seen that clockwise rotation of the upper supporting leg 322 about the axis defined by the second (supporting leg) coupling point 512 increases the height of the patent support apparatus 310 and the first (supporting leg) coupling point 511.

In other words, the patient support apparatus 310 is configured to rotate, with respect to the support structure 320, about a principal axis (e.g. a pitch axis). The rotation mechanism controls this rotation. The height adjustment mechanism controls the height of the principal axis. These adjustments can be controlled together in order to define an 'effective' axis of rotation. For example, by rotating the patient support apparatus 310 about the pitch rotation axis and also increasing the height of the pitch rotation axis at the same time, in the reference frame of the treatment room the effect is that the patient support apparatus 310 rotates about an effective axis of rotation which does not align with the principal rotation axis defined by the point 511 at which the patient support apparatus 310 and the support structure 320 are coupled to one another. By controlling these degrees of freedom appropriately, the effective axis of rotation can be made to pass through the isocentre. By controlling pitch, yaw, and roll, it is possible to define the isocentre as an effective point of rotation about which the patient support surface can rotate.

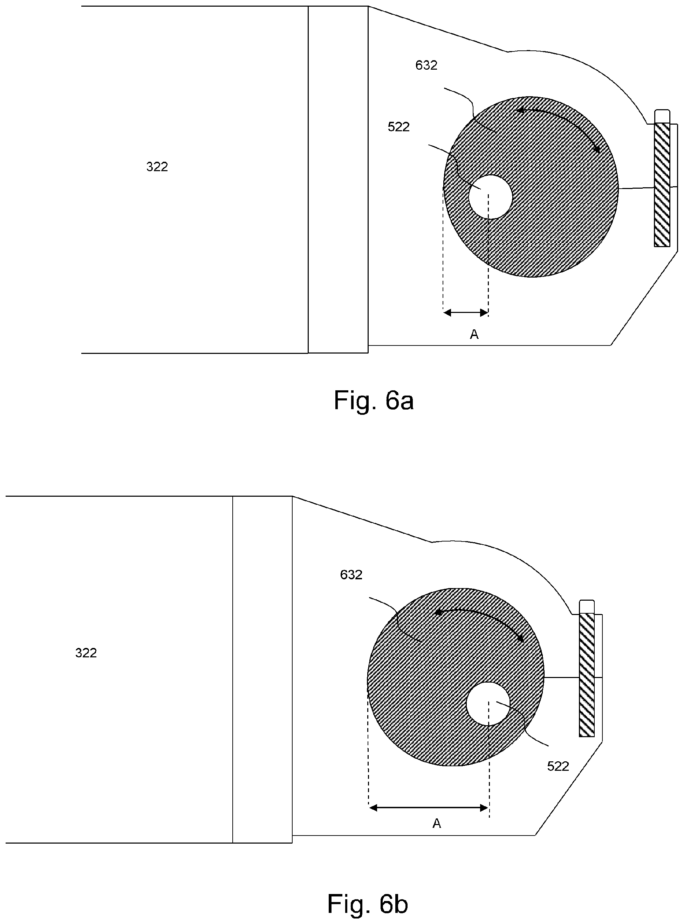

Figure 5 depicts a close-up version of the rotation mechanism depicted in figures 2a-c, 3a,b and 4a. Figures 6a and 6b show a cross-section through the joint which defines the second drive member coupling point 522.

The drive member 322 is rotationally coupled to the upper coupling element 325 at a first coupling point 521 and rotationally coupled the support element 324 at a second coupling point 522 as described above. The drive member 322 may thus rotate about a first axis (defined by the first coupling point 521) and a second axis (defined by the second coupling point 522). This second axis may be referred to as a 'common' axis of rotation as will be described below.

The actuation mechanism comprises a motor 612 configured to propel a drive nut 616 along a threaded shaft 618. The motor 612, drive nut 616 and threaded shaft 618 form part of a linear actuator arrangement. The linear actuator arrangement may be a ball screw actuator, the functionality of which will be understood by the skilled person. The linear actuator arrangement further comprises a housing 614. The housing 614 is rotationally coupled to the drive member 322. The housing 614 comprises one or more spindles 615 which fit into corresponding apertures within spindle holders 624 fixedly attached to the drive member 322. This arrangement allows the linear actuator arrangement, including the motor 310 and threaded drive shaft 618, to rotate with respect to the drive member 322 about an axis defined by the spindle(s) 615 and spindle holder(s) 624. This rotation axis is perpendicular to the longitudinal axis of the drive member 322 and parallel with each of the rotational axes described above in relation to the principal axis and the various coupling points.

The rotation mechanism further comprises a motion converter 632. The motion converter 632 is rotationally coupled to the drive member 322, and to the support element 324, at the second (drive member) coupling point 522. Thus, both the drive member 322 and the motion converter 632 may rotate with respect to the support element 324 about a common axis of rotation defined by the second drive member coupling point 522. The motion converter 632 is configured to convert linear motion of the linear actuator, and in particular the linear motion of the drive nut 616 along the threaded shaft 618, into rotatory motion about this common axis of rotation.

To achieve this conversion, the motion converter 632 comprises a crank arm 634, or link arm, which is coupled to the drive nut 616. The crank arm 634 is rotationally coupled to the drive nut 616 by virtue of one or spindles extending out from the drive nut 616, which fit in corresponding apertures (or spindle holders) in the crank arm 634. The various features are arranged and configured such that linear movement of the drive nut 616, controlled by the motor 612, causes rotation of the motion converter 632 about the common axis of rotation.

The motion converter 632 passes through an aperture in the drive member 322. The aperture extends in a direction defined by the longitudinal axis of the drive member, and may be circular in shape. The motion converter 632 is mounted eccentrically with respect to the common axis of rotation. In the implementation depicted in figure 5, the support element 324 comprises an axle at 522 which defines the location of the common axis of rotation, and the motion converter 532 is mounted eccentrically with respect to this axle 522. This mounting is shown in detail in figures 6a and 6b. As will be appreciated from the accompanying description of these figures, the motion converter 532 is eccentrically mounted with respect to the axle such that rotation of the motion converter 532 about the axle causes linear movement of the drive member 322 in the manner discussed elsewhere herein.

Figures 6a and 6b depict the joint at which the motion converter 632 and the drive member 322 are coupled to the fixed axle of the support element 324. Both the drive member 322 and the motion converter 632 may rotate about this axle and thus the drive member 322 and the motion converter 632 share a common axis of rotation. Rotation of the motion converter 632 about this axis is controlled by the motor 612, via its control of the movement of the drive nut 616 along the threaded shaft 618.

As is described elsewhere herein, the rotation mechanism is configured to adjust a distance between the two rotational coupling points of the drive member 322. This can be achieved by adjusting the distance A depicted in figures 6a and 6b. Figure 6a shows the distance A toward, or at, its minimum. Figure 6b shows the distance A toward, or at, its maximum. The position of the axle defines the second, or lower, coupling point 522 of the drive member 322. By rotating the motion converter 632 about the common axis it shares with the drive member 322, the amount of the motion converter 632 which is interposed between the first and second coupling point of the drive member 322 is adjusted. This is achieved by virtue of the eccentric mounting of the motion converter 632 with respect to the axis at 522.

This particular implementation of the rotation mechanism is advantageous. The motion converter 632 is eccentrically mounted with respect to a fixed axle of the supporting structure 320, and the drive member 522 is also rotationally mounted to the axle to define a rotation axis common to both the drive member 322 and the motion converter 632. This arrangement allows the linear movement of the drive member 322 along its longitudinal length (which movement controls the rotation of the patient support apparatus) to be controlled via a linear actuator arrangement; however, crucially, the full load of the patient support apparatus is not borne by the linear actuator arrangement. Thus, a more stable arrangement is provided which is less prone to damage and issues causes by mechanical wear.

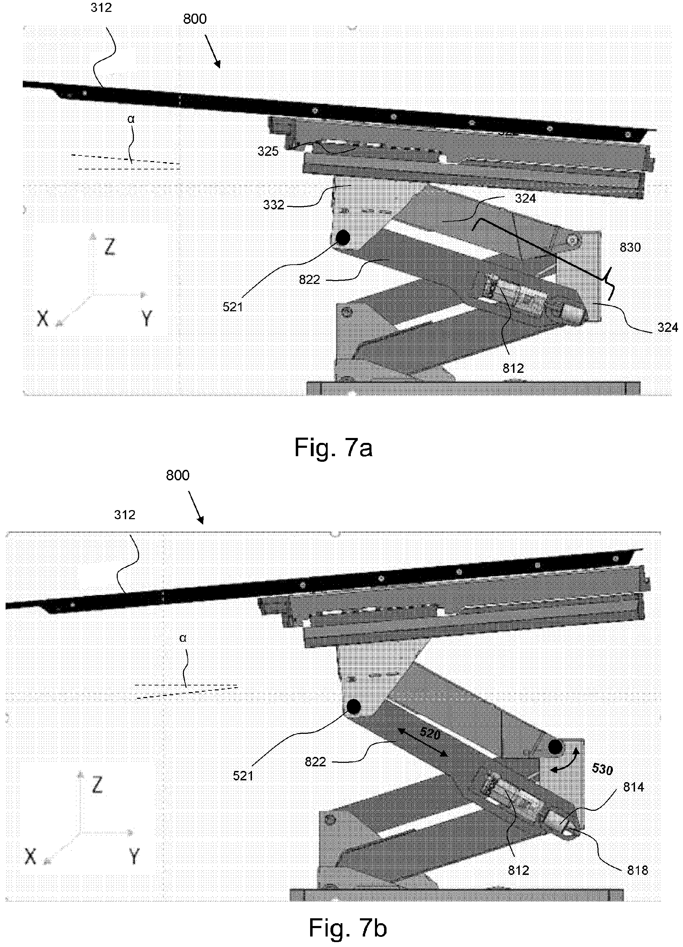

Figures 7a and 7b depict a patient positioning apparatus 800 comprising an alternative implementation of the rotation mechanism. Other than the rotation mechanism, the arrangement 800 of figures 7a and 7b is similar to the arrangement 300 depicted in figures 2-c and 3a,b, and like reference numerals are used to refer to like features. Figure 7a depicts the apparatus 800 at or toward its maximum positive tilt angle α, and figure 7b depicts the apparatus 800 at or toward its maximum negative tilt angle α. Figures 6a and 6b show a cross-section through the joint which defines the second drive member coupling point 522 in this implementation. The actuation mechanism is not shown in figures 6a and 6b.

The rotation mechanism comprises a drive member 822 and an actuation mechanism 830. The actuation mechanism 830 achieves the same effect as rotation mechanism 330 described above. In particular, the actuation mechanism 880 is configured to move the drive member 822 so as to impart a force to an underside of the patient support apparatus and thus tilt the patient support apparatus with respect to the support structure. The actuation mechanism 830 comprises a motor 812, a drive nut 814, and a threaded shaft 818. The actuation mechanism 830 may be a high load ball screw actuator or roller screw actuator, or may be a suitable high load hydraulic actuator.

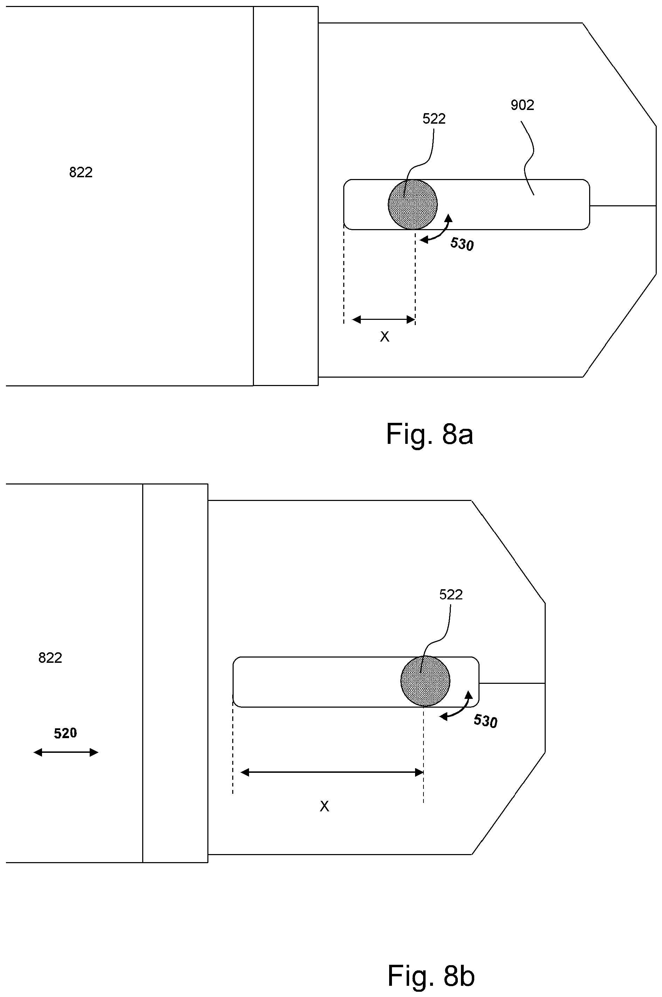

The drive member 822 comprises an aperture 902. The aperture 902 extends in a direction defined by the longitudinal axis of the drive member 822. The motor 812, drive nut 814, and threaded shaft 818 are attached to, and may be integral with, the drive member 822. The motor 812 is rigidly affixed to the drive member 822 and is configured to move with the drive member 822. The threaded shaft 818 extends parallel with the aperture 902, i.e. in a direction parallel with the longitudinal axis of the drive member 822. The threaded shaft 818 may extend into the aperture 902. The threaded shaft 818, drive nut 814 are not shown in figures 8a, 8b.

An axle extends from the support element 324 to define the location of the lower (or second) drive member coupling point 522. The axle extends into the aperture 902 and is rotationally coupled to the drive nut 814 to define an axis about which the drive nut 814, and hence drive member 822, may rotate with respect to the support element 324. The drive nut 814 may also be referred to as a drive member or drive element.

The drive element 814 is rotationally coupled to the support structure, but has no translatory of degrees of freedom with respect to the support element 324. The drive element 814 is rotationally attached to an axle which cannot move with respect to the support structure. The motor 812 is configured to control the position of the drive nut 814 along the threaded shaft 818. Because the axle is coupled to the drive nut 814 in or though aperture 902, actuating the motor 812 has the effect of controlling the location of the axle in the aperture 902. Actuating the motor 812 in a particular direction has the effect of pushing the motor 812, and with it the drive member 822, away from the drive nut 814. Actuating the motor 812 in the opposite direction has the effect of pulling the motor 812, and with it the drive member 822, toward the drive nut 814.

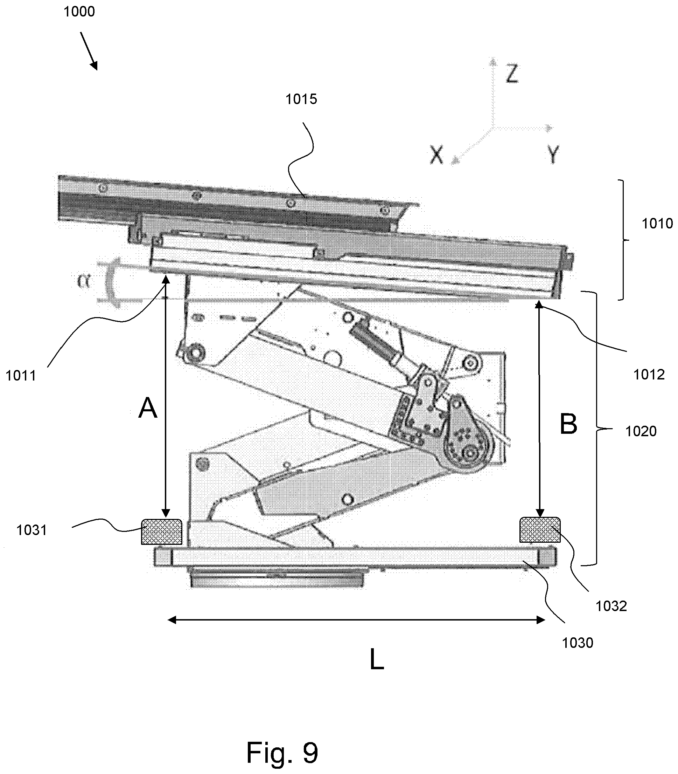

This functionality can be appreciated upon inspection of figures 9a and 9b. The actuation mechanism is not shown in these figures, but is configured to control the movement of the axle at 522 within the elongated aperture 902. Therefore, the actuation mechanism is configured to control an adjustable distance, A, in a manner similar to the actuation mechanism 530 depicted in figures 7a, 7b.

As the actuation mechanism 330 adjusts the distance between the first and second coupling point, the distance between the first coupling point and the support element 324 is also adjusted. By increasing the distance between the first and second coupling points, the first coupling point, and thus the coupling element, is pushed away from the support element 324 of the support structure 320. Conversely, by reducing the distance between the first and second coupling points, the first coupling point, and thus the coupling element 325, are pulled toward the support element 324 of the support structure 320.

As with actuation mechanism 330, the actuation mechanism 830 is configured to adjust the distance between the first and second drive member coupling point. Adjusting this distance in one of the two possible directions moves the drive member 822 in that same direction. By virtue of this arrangement, the rotation mechanism is configured to impart a force, via the drive member 822, to an underside of the patient support apparatus to thereby rotate the patient support apparatus with respect to the support structure.

Still further alternatives of the rotation mechanism are possible. In a simple alternative embodiment to the implementations described above, the drive member may have an adjustable length. The actuation mechanism is configured to adjust this length in order to impart a force to an underside of the patient support apparatus. This may be achieved using a telescoping linear actuator. For example, a first, radially outer element of the drive member may be coupled to one of the support element or the coupling element, and a second, radially inner element of the drive member may be coupled to the other of the support element or the coupling element. The actuation mechanism is configured to control the degree to which the inner element extends out from the outer element in a manner that will be known to the skilled person, who will be familiar with different forms of linear actuators. Thus, by controlling the length of the drive member, the distance between the first, upper drive member coupling point and the second, lower coupling point of the drive member can be controlled in a manner similar to that described above. Control of this distance allows a force to be imparted to the underside of the patient support apparatus in order to tilt the patient support apparatus in the manner described elsewhere herein.

The patient positioning apparatus may further comprise a swivel mechanism, which is embeddable within a floor of a treatment room, and which is configured to rotate the patient positioning apparatus with respect to the treatment room. This may be described as a yaw rotation. This mechanism is in accordance with known mechanisms and need not be discussed further herein.

It is worth noting that, while the implementations described in detail herein control a tilt in the form of a pitch rotation, the skilled person will appreciate that the presently disclosed rotation mechanism may effect rotation about any of a number of different axes, and in particular may control a roll rotation if the patient support apparatus is correctly oriented with respect to the support structure.

A general aim of the present disclosure is to provide a space-efficient and compact apparatus. By providing a rotation mechanism in the manner disclosed herein which is attached to, and supported by, the support structure, a number of benefits are provided. Mechanical 'pinch points' no longer limit the maximum degree of tilt, and the need for a large separation between a base plate and a patient support surface is removed. Thus, the hop-on height is advantageously reduced. The attachment of the rotation mechanism to support structure which is configured to support the patient support apparatus, e.g. in the support legs of the apparatus, means that the rotation mechanism and in particular its actuation mechanism is easier to access, service and repair. Linear movement of the patient support surface with respect to a patient support base is facilitated as the entire patient support apparatus is lighter and less bulky, and the need to translate not only the patient support apparatus but also the rotation mechanism as part of this movement is removed.

Having the support structure support the weight of the patient support apparatus, with a separate rotation mechanism which controls rotation with respect to that support structure, means that the rotation mechanism itself need not directly support a heavy load. This reduces mechanical wear and tear, reduces chances of breakdown, and increases the longevity of the apparatus.

### Sensor arrangement

Also disclosed herein is a patient positioning device which comprises a tiltable patient support apparatus and a sensor arrangement configured to determine a degree of tilt. Adjusting the position of a patient using a patient positioning apparatus or device is common in various fields of medicine. For example, the patient may be tilted prior to or during radiotherapy treatment in order to adjust the position of the patient with respect to a source of therapeutic radiation, and thereby to adjust the dose distribution in the patient's body. For example, a treatment plan may call for the adjustment of the patient via tilting the support surface in order to reduce the dose applied to a particular region of healthy tissue.

For safety reasons, it is very important to be able to determine the position of the patient prior to and during treatment. Actuator arrangements may be used to adjust the height of a patent support surface, or the degree to which the patient is tilted, and the traditional way to measure the height and/or tilt is to place encoders on all the motors and joints of the patient positioning device. The signals from each of these encoders can be used to indirectly measure the angle and height of the patient table. This prior process requires the processing of several measurements, through the mechanical structure, to finally reach the resulting tilt angle. Every step in the determination introduces inaccuracies, due to measurement errors and structural stiffness, that will add uncertainty to the result.

The present application seeks to address these and other disadvantages in the prior art by providing a patient positioning device or apparatus comprising an improved sensor arrangement.

Disclosed herein is a patient positioning device which comprises a tiltable patient support apparatus and a sensor arrangement. The sensor arrangement comprises a processor and at least two sensors communicatively coupled to the processor. The sensors are spaced from one another, and each sensor is configured to measure a distance between an underside of the patient support apparatus and a respective fixed location. The sensors may be located at the fixed locations; for example, the sensors may be incorporated into a base of the patient positioning device such that the sensors measure a distance from the base to the underside of the patient support apparatus. Based on these signals, the processor can determine the degree of tilt of the patient support apparatus.

By measuring these distances directly, and along a line of sight of the sensors, the measurement of tilt is greatly simplified. It is possible to use a simple ratio of the measured distances in order to determine the degree of tilt. In this manner, fewer errors, for example due to sensor error margins and structural tolerances, are introduced to the measurement in comparison with prior techniques which measure the tilt using indirect measurements.

Ensuring the accuracy of the measured tilt angle is important. It has been found that a rotational error of 3° reduces coverage of a clinical target volume in brain tumors treated with intensity modulated radiotherapy from 99.3 to 97.0%. In other words, when the patient positioning device is used in conjunction with a radiotherapy device, improving the accuracy of the tilt measurement means also improves the accuracy and efficacy of radiotherapy treatment.

Figure 9 shows a patient positioning apparatus / device 1000 according to the present disclosure. The positioning device 1000 may be substantially as described elsewhere herein, such as the examples of figure 3 or figure 7, or may take another form. The device 1000 comprises a patient support apparatus 1010 comprising a patient support surface 1015. The device 1000 further comprises a base 1030, and support structure 1020 to support the patient support apparatus 1010 above the base 1030. A patient may lie on the patient support surface 1015 when the patient positioning device 1000 is in use.

The device 1000 comprises a rotation mechanism, or system, which may take any appropriate form, for example the form described elsewhere herein. With reference to figure 10, the rotation system is configured to tilt the patient support apparatus 1010, and thereby the patient support surface 1010, about a pitch axis 1110. Alternatively or additionally, the rotation system may be configured to tilt the patient support apparatus 1010, and thereby the patient support surface 1015, about a roll axis 1120. The rotation system may therefore be configured to cause and control rotation of the patient support apparatus by one or both of pitch and roll. The rotation system may be comprised of separate pitching and rolling mechanisms. The exact form of the rotation mechanism is not important, and several implementations are envisaged.

The device 1000 further comprises a sensor arrangement. The sensor arrangement comprises a plurality of sensors. The plurality of sensors comprises at least a first sensor 1031 and a second sensor 1032. The sensor arrangement comprises a processor (not shown), and the first and second sensors 1031,1032 are communicatively coupled to the processor. The sensors 1031,1032 send signals to the processor, and based on these signals the processor is configured to determine a degree of tilt α of the patient support apparatus 1010.

The first sensor 1031 is configured to provide signals indicative of a first distance, A, between a first region 1011 of the underside of the patient support apparatus 1010 and a first fixed location underneath the patient support apparatus 1010. Similarly, the second sensor 1032 is configured to provide signals indicative of a second distance, B, between a second region 1032 of the underside of the patient support apparatus 1010 and a second fixed location underneath the patient support apparatus 1010. Distances A, B can be described as vertical distances.

The sensors produce signals indicative of the distances A,B. The value of A can be derived from signals produced by the first sensor. For example, in the case of an optical sensor, the signals produce might relate to the intensity of light which has been reflected back from the underside of the patient positioning apparatus. The value of A can be derived by reference to calibration data, which in a simple form may be a look-up table relating light signal intensity to distance values.

Reference is made to fixed locations. In some implementations the base may be configured to swivel or rotate, thereby rotating the entire patient positioning device. In such an implementation, the term 'fixed location' or 'fixed position' means fixed with respect to the base, rather than fixed with respect to the treatment room.

In the implementation depicted in figure 9, the first sensor 1031 is located at the first fixed location such that the distance A is a distance between the first sensor 1031 and the first region 1031 of the underside of the patient support apparatus 1010. The second sensor 1032 is located at the second fixed location such that the distance B is a distance between the second sensor 1032 and the second region 1032 of the underside of the patient support apparatus 1010.

The first and second fixed locations may be located substantially at, or on, a base 1030 of the patient positioning device 1000. In the implementation of figure 9, the sensors are positioned on the base 1030 of the device 1000 such that distances A, B can be thought of as the distance between the regions 1011 and 1012, of the underside of the patient support apparatus 1010 and the base 1030, accounting for any systematic 'additional distance' introduced by the positioning of the sensors 1031, 1032, for example the height of the sensors 1031, 1032 above an upper surface of the base 1030.

The sensors 1031, 1032 may take many forms. For example, the sensors 1031, 1032 may be optical sensors such as optical distance sensors. Such sensors are known to the skilled person and typically make use of pulsed light. The strength of the returned signal from a target surface is indicative of the distance between the sensor and the target surface. Alternatively, the time taken for a beam to be reflected rom a target surface and return to the sensor may be used to determine the distance between the sensor and the target surface. In the implementation depicted in figure 9, the underside of the patient support apparatus 1010 represents the target surface for the optical sensors. However, equivalently, the sensors may be located on the underside of the patient support apparatus 1010, with the target surfaces being fixed positions underneath the support apparatus, for example fixed positions located on the base. The optical sensors may be used in conjunction with appropriately placed mirrors / light reflectors / targets to increase the efficiency of returned light, for example placed at the first and second region 1011, 1012, however mirrors / light reflectors are not required. The sensors may be triangulation laser sensors.

The sensors may be any suitable distance measurement sensors and may take other forms, and for example may take the form of draw string or draw wire sensors. A draw wire sensor comprises a main body and a wire rolls up like around a reel in the main body. The reel has a rotational encoder attached that counts the turns and hence the length of wire pulled out from the main body. The main body of the draw wire sensors may be coupled with the underside of the patient positioning apparatus 1010, with the wire extending downward toward a hook which is fixedly attached at one of the fixed locations. The displacement of the wire along the line joining a region of the underside of the patient positioning apparatus 1010 and the fixed location is then indicative of the distance between these points. Alternatively, the main body of the sensor may be positioned at the fixed position, with the opposing end of the wire fixedly attached to the underside of the patient positioning apparatus 1010. In other words, the string may be attached to the tilting surface with the sensor body attached to the fixed base 1030.

Another type of sensor which may be used is a linear travel sensor. Linear travel sensors may comprise, for example, a ring that travels along a rod. The ring's linear motion along the rod can be converted into electrical signals.

The sensors, be they draw-string / draw-wire, optical, or another type of sensor, may undergo some form of calibration routine. The calibration will be standard in nature for the type of sensor used, for example to calibrate the sensors for their linearity error to make them as accurate as possible, and to set the readings which correspond to zero tilt angle and/or zero height. Such calibration is known to the skilled person.

As can be seen in the implementation depicted in figure 9, the sensors 1031, 1032 are spaced from one another. The sensors in figure 9 are spaced from one another so as to enable the determination of a degree of tilt α in the form of a rotation about the pitch axis 1110. As can be seen in figure 9, the degree of pitch α may be defined as the degree to which the patient support apparatus 1010, and with it the patient support surface 1015, has been tilted about the pitch axis 1110. The degree of pitch α may be defined in multiple ways, though it is not necessarily important to define the angle in a particular way; the important thing is that the system is configured to determine a change in angle α between different tilt positions. In an implementation, the degree of pitch α can be described as the angle which the patient support surface 1015 makes with a horizontal plane, or equivalently the angle which the patient support surface 1015 makes with the floor of the treatment room. α may also be referred to as the pitch angle.

The first and second fixed locations are separated by a separation distance L. When the sensor arrangement is configured to measure a pitch angle α, the separation distance L extends parallel to the roll rotation axis 1120. In other words, the first and second fixed locations are separated by a separation distance L along an axis parallel to the roll axis 1120. In the implementation depicted in figure 9, the first sensor 1031 and second sensor 1032 are positioned directly underneath and aligned with the roll axis 1120.

The patient positioning apparatus 1015 can be thought of as having a major axis and a minor axis. At zero pitch tilt, the major axis is parallel with and may align with the roll axis 1120, and at zero roll tilt, the minor axis is parallel with and may align with the pitch axis 1110. The pitch angle α may be defined by an angle formed between the major axis of the patient positioning surface 1015 and the roll axis 1120. The roll angle β may be defined by an angle formed between the minor axis of the patient positioning surface 1015 and the pitch axis 1110. The discussions relating to axes and pitch/roll angles in relation to figure 10 apply similarly to all implementations discussed herein.

The first and second fixed locations are separated in a direction parallel with this major axis, measured when the patient positioning apparatus is at zero tilt, such that the processor is configured to determine, based on signals from the sensors, the degree of pitch of the patient support apparatus 1010.

Another way of describing the major axis of the patient positioning apparatus 1000 is as a length axis. Using this terminology, the patient positioning surface 1015 comprises a length defining, at zero tilt, a length axis. The length axis is parallel with and may align with the roll rotation axis 1120. As can be seen in figure 9 with reference to figure 10, the first and second fixed locations are separated in a direction along the length axis / major axis / roll axis by a distance L. In a specific implementation, L may be, for example, substantially 1m.

The processor is configured to determine, based on signals from the sensors, the degree of pitch of the patient support apparatus 1010. The degree of pitch, i.e. the pitch angle α, can be calculated using the following formula: $α = {tan}^{- 1} \left(\frac{A - B}{L}\right) .$

It will be appreciated that the determination of the pitch angle α using this simple formula relies on significantly fewer factors than previous measurement techniques, and thus fewer errors are introduced into the calculation.

Accordingly, disclosed herein is a method of determining a degree of tilt of the patient support apparatus 1010. In its simplest form, the method comprises determining the degree of tilt of the patient support apparatus 1010 based on signals received from the sensors 1031, 1032. The method may comprise determining, or deriving, a first value based on signals received from the first sensor 1031, determining, or deriving, a second value based on signals received from the second sensor 1032, and determining the degree of tilt based on a ratio of the first and second value. The values may be the distances A and B. If the sensors are not located the same distance from the underside of the patient support apparatus 1010 at zero tilt, e.g. if A≠B when α =0, then the known height difference between the first fixed location and the second fixed location is incorporated into the calculation in order to calculate α.

This method can be embodied on a computer-readable medium, which may be a non-transitory computer medium, which comprises computer executable instructions which, when performed by the processor, cause the processor to carry out the method.

The sensors in figure 9 are separated along a length axis of the couch, or equivalently along the roll rotation axis of the couch. This allows the sensors to measure the pitch angle. However, the first 1031 and second sensor 1032 may instead be separated in a direction parallel with a width axis of the couch, or equivalently along the pitch rotation axis of the couch. This can also be thought of as a separation in a direction parallel with the minor axis of the patient positioning apparatus 1000. This allows the sensors to measure the degree of roll, or roll angle, of the patient support apparatus 1010.

In other words, the patient positioning surface may comprise a width defining, at zero tilt, a width axis. This width axis is parallel or else aligns with the pitch rotation axis. By separating the first and second fixed locations along the width axis, the processor may use signals from the first and second sensors to determine the degree of roll of the patient support apparatus 1010.

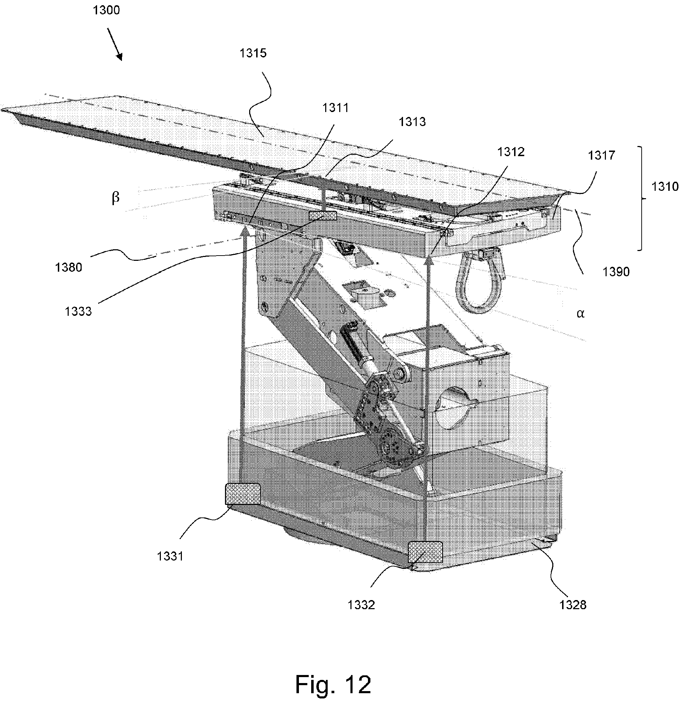

Generally speaking, by using just two sensors as depicted in figure 9, the height of the patient support apparatus base can be determined at any point along a line joining the first region 1011 and the second region 1012. However, in some implementations, the patient support base is configured to tilt in one degree of freedom (e.g. pitch), with the other tilting degrees of freedom of the support surface 1015 being effected by rotation mechanisms which do not cause tilting or movement of the patient support base. For example, the roll mechanism may be incorporated into the patient support surface and may effect roll tilting of the patient support surface 1015 with respect to the patient support base (such an implementation is depicted in figure 12) . In such an implementation, in which the regions of the underside of the patient support base can only rotate about one tilting axis, it is possible to determine the height of any region of the underside of the patient support base using just two sensors In implementations in which the tilting of the patient support base is not limited to one degree of freedom, to ensure the height of the centre of the patient support apparatus can be determined, the sensors may be placed such that the centre point of the patient support apparatus 1010 lies along, or above, this line.

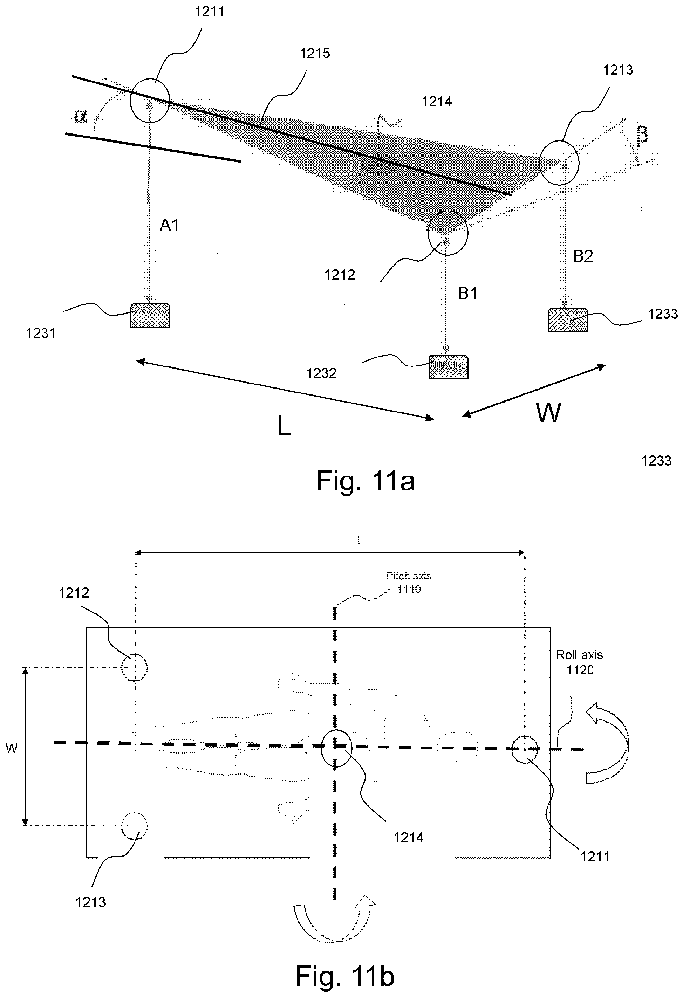

Another implementation of the present disclosure is depicted in figures 11a and 11b. Figure 11a shows a plane 1215 which can be defined using signals from a plurality of sensors. Figure 11b is a view of a patient positioning apparatus from above and shows the patient support surface supporting a patient. The positions of four regions of an underside of a patient support apparatus / patient support surface. The regions 1211, 1212, 1213, 1214 are located on the underside of the apparatus, i.e. on a lower side opposite an upper side of the patient support surface, though it will be appreciated that their positions can nevertheless be indicated in a top down view of the patient support apparatus in the manner shown in figure 11b. In figure 11b, the table is shown at zero tilt.

In this implementation, a first sensor 1231 is positioned at a first fixed location and is configured to provide signals to a processor which are indicative of a distance A1 between the first fixed location and a first region 1211 of the underside of the patient positioning apparatus. A second sensor 1232 is positioned at a second fixed location and is configured to provide signals to the processor which are indicative of a distance B1 between the second fixed location and a second region 1212 of the underside of the patient positioning apparatus. The second fixed location is separated from the first fixed location by a distance L as measured in a direction parallel with the roll axis / length axis of the patient support apparatus in the manner shown in figure 11b. A third sensor 1233 is positioned at a third fixed location and is configured to provide signals indicative of a distance B2 between the third fixed location and a third region 1213 of the underside of the patient positioning apparatus. The third fixed location is separated from the second fixed location by a distance W as measured in a direction parallel with the pitch axis / width axis of the patient support apparatus. The first region 1211, second region 1212 and third region 1213 define a plane 1215 which describes the position in space of the patient support apparatus.

By arranging a plurality of sensors in the manner described with respect to figures 11a and 11b, the degree of tilt of the patient support apparatus in the form of both pitch and roll can be determined. In figure 11a, the degree of pitch, i.e. the pitch angle, is denoted by α. The degree of roll, i.e. the roll angle, is denoted by β. The processor is configured to determine, based on signals from the first, second and third sensors, the degree of pitch and the degree of roll of the patient support apparatus 1010. Processing performed by the processor may make use of appropriate and simple formulae, and in particular the processing may make use of relatively simple trigonometric calculations. In simple summary, the sensors allow you to measure the sides of a triangle, and compare with the known sides of the triangle from which is it possible to calculate an angle of interest. It will be understood that these calculations can be adjusted depending on the angles of interest and depending on the placement of the sensors. For example: $α = {tan}^{- 1} \left(\frac{A 1 - \left(\frac{B 1 + B 2}{2}\right)}{L}\right)$ $β = {tan}^{- 1} \left(\frac{B 2 - B 1}{W}\right)$

In this implementation, the processor may be further configured to determine the height of the patient support surface, for example via determining the height of a fourth region of the underside of the patient support surface.

The height of the patient support apparatus 1010 can be determined using the distance information provided by each of the first, second, and third sensors. If height is defined as the distance between an underside of the patient support surface and the base, then if the fixed locations are located at the base of the patient positioning device, the distances measured by the sensors are height measurements. For example, in the implementations depicted in figures 9 and 11a, 11b, the sensors form part of, and/or are embedded within, the base of the patient positioning device. With reference to the distances depicted in figure 11a, distance A1 can be thought of as the height of the first region 1031, B1 can be thought of as the height of the second region 1032 and B2 can be thought of as the height of the third region.

The distance / height information, in conjunction with knowledge of the relative positions of the sensors / fixed locations, can be used to define a co-ordinate in space for each of the three different regions of the underside of the patient support apparatus 1010. The three co-ordinates define a plane which describes the position in space of the patient support apparatus 1010. Using simple geometry, the co-ordinates, and thus height, of any point on the plane can be determined. In this way, the height of a fourth region of the underside of the patient support surface can be determined based on the distances measured by the three sensors.

The height may be defined in a number of ways, for example the distance between the fourth region 1214 and the base, or the floor of the treatment room. The desired height value may relate to the height of the centre of the patient support apparatus, and therefore the fourth region 1214 may be a central region of the patient support apparatus. The height of any point on the plane 1215 formed by the three measurements A1, B1, and B2 can be determined.

In another implementation (not shown), the patient support apparatus may be configured to rotate about a yaw axis that extends into the plane of the diagram shown in figure 11b. To measure a yaw angle, a sensor is provided which is tilted, or else mounted horizontally, with respect to the patent support apparatus. This is in contrast with the sensors described thus far, which are vertically mounted. If the yaw axis of rotation is fixed, one horizontally oriented sensor may be configured to determine the yaw rotation. Reading the increase in distance gives the side of the triangle from which the yaw angle can be calculated in a manner similar to that described above. If the patient support surface is configured to provide a horizontal displacement of the apparatus and the yaw axis, a second horizontally mounted sensor is provided to distinguish between what is a side movement and what is a rotation. These sensors sit outside the patient positioning system on a fixed surface relative to the treatment room. The same principles described above can be employed, though with horizontal rather than vertical measurements.

Figure 12 depicts a patient positioning apparatus 1300. The patient positioning apparatus 1300 depicted is similar in form and functionality to the patient positioning apparatuses described elsewhere herein, and in particular the pitch rotation mechanism takes the form depicted in any of figures 2a-6b. However, it will be appreciated from the following description that the pitch rotation mechanism may take any suitable form. The patient positioning apparatus 1300 also comprises a roll rotation mechanism incorporated into the patient support apparatus 1310.

The patient positioning apparatus comprises a patient support surface 1315 configured to rotate about a roll axis 1390 with respect to a patient support base or base structure 1317. This rotation is controlled by a rotation mechanism positioned between the patient support surface 1315 and the patient support base structure 1317.

The patient positioning apparatus further comprises a rotation mechanism, which may be similar in form to that described above in relation to figures 2a-6b, configured to rotate the patient positioning apparatus about 1310 about a pitch rotation axis 1380 with respect to a positioning apparatus base or base structure 1328. This rotation is controlled by a rotation mechanism positioned between the patient support apparatus 1310, and in particular the patient support base or base structure 1317, and the base 1328. In this implementation the mechanical axes of pitch 1380 and roll 1390 are "stacked", and/or positioned in different layers, with respect to one another. In other words, these rotational axes are at different heights.

A first sensor 1331 and a second sensor 1332 are positioned to measure a height of a first region 1311 of the underside of the patient support base 1317, a height of a second region 1312 of the underside of the patient support base 1317, and determine a pitch angle α in the manner described above with respect to figure 9. These sensors placed in a lower 'layer', e.g. between the apparatus base 1328 and patient support base 1317, are used to calculate the height of the patient support apparatus and the pitch angle α.

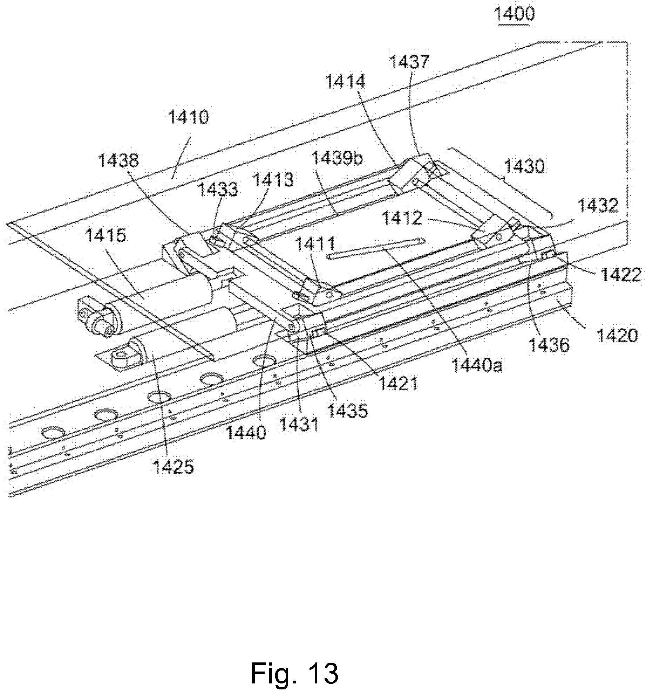

The patient support base in the specific implementation shown is configured to be rotated about one axis of rotation with respect to the fixed location of the sensors 1331, 1332 positioned on the base 1328. This axis is the pitch rotation axis. The roll rotation mechanism is positioned between the patient support base and the patient support surface, and for example may take the form described with respect to figures 13 to 15. This mechanism does not control movement of the patient support base, but instead controls movement of the patient support surface 1315 directly. The yaw rotation is controlled by the swivel mechanism, which rotates the entire device 1300, and with it the sensors 1331, 1332 and the patient support apparatus 1310. Because sensors 1331 and 1332 are measuring a distance to the underside of a base which itself is constrained to only rotate about one axis of rotation with respect to the sensors, it is possible to position the two sensors 1331, 1332 at any position underneath the patient support base and calculate the height of any position of the underside of the patient support base / apparatus. In particular, the two sensors can be moved anywhere in a parallel direction to the pitch rotation axis, and the sensors do not have to be positioned directly under the roll axis in order to determine the height of the central region of the patient support apparatus 1310. In other words, by restricting the mechanics it is possible to calculate the height anywhere on the underside of the patient support apparatus 1310 since this surface will always be parallel to pitch rotation axis 1380, which itself is always horizontal.

The apparatus further comprises a third sensor 1333 positioned between the patient support base 1317 and the patient support surface 1315. The third sensor is similar in form and functionally to those described elsewhere herein, and is configured to measure a distance from a third fixed location to a region 1313 of an underside of the patient support surface 1315. The third sensor 1333 may be positioned at the third fixed location. Signals received from the sensor 1333 positioned in this 'upper layer', i.e. between patient support base / base structure 1317 and patient support surface 1315, may be used to calculate the roll angle β. Accordingly, the third sensor 1333 may be referred to as a roll angle sensor or a roll rotation sensor. A single sensor in this layer may be used to determine the roll angle β by virtue of a simple calibration process which creates a mapping between measured height values and tilt angles.

A method of determining a degree of tilt of the patient support apparatus is also disclosed herein. The method comprises determining the degree of tilt of the patient support apparatus based on signals received from the sensors. A first value is calculated based on signals received from the first sensor, and a second value is calculated based on signals received from the second sensor. There may be some processing to account for any systematic height or distances, in particular if the first and second sensors are not placed in the same horizontal plane. The method comprises determining the degree of tilt based on a ratio of the first and second value. For implementations in which the patient support apparatus comprises a third sensor configured to provide signals indicative of a third distance between a third region of the underside of the patient support apparatus and a third fixed location underneath the patient support apparatus, the method may further comprise determining a third value based on signals received from the third sensor;
determining the degree of pitch of the patient support apparatus based on the ratio of the first and second value, and determining the degree of roll of the patient support apparatus based on the ratio of the second and third value.

The method may be used with a patient support base structure and a patient support surface, where the patient support surface is tiltable about a roll rotation axis with respect to the base structure, and wherein the apparatus comprises a roll rotation sensor coupled to the processor and configured to provide signals indicative of a distance between a first region of the underside of the patient support surface and a fixed location between the patient support base structure and the patient support surface. In such an implementation, the method may also comprise determining a degree of pitch of the patient support apparatus based on signals received from the first and second sensors, and a degree of roll based on signals received from the roll rotation sensor.

The present disclosure relates to the use of a relatively small number of sensors which are able to accurately determine a degree of tilt of a patient support apparatus comprising a patient support surface. The sensor arrangement of the present disclosure provides a simple, cost effective, and accurate measurement of tilt.

By using sensors configured to provide signals indicative of distances between regions of the underside of the patient support apparatus and fixed locations underneath the patient support apparatus, a direct measurement of the height, and thus position, of the patient positioning surface can be obtained. The degree of tilt of the support apparatus, whether it be a pitch or a roll angle, can be determined by making use of simple ratios of the measured distances. Thus, the need to infer the tilt angle and/or height of the patient positioning surface via a complex process involving multiple indirect measurements is removed. Positioning and configuring the sensors in this way removes measurement errors and structural stiffness and thus reduces uncertainty in the result.

By making use of signals provided by three appropriately positioned sensors, the degree of both pitch and roll can determined, in addition to the height of any desired location of the patient positioning apparatus or patient positioning surface.

In particular disclosed arrangements, a patient positioning apparatus for a medical device comprises a tiltable patient support apparatus and a sensor arrangement, the sensor arrangement comprising a processor, and a first and a second sensor communicatively coupled to the processor, wherein the sensors are spaced from one another, the first sensor being configured to provide signals indicative of a first distance between a first region of the underside of the patient support apparatus and a first fixed location underneath the patient support apparatus, and the second sensor being configured to provide signals indicative of a second distance between a second region of the underside of the patient support apparatus and a second fixed location underneath the patient support apparatus, wherein the processor is configured to determine, based on signals from the sensors, a degree of tilt of the patient support apparatus.

Optionally, the first and second fixed locations are located substantially at a base of the patient positioning device.

Optionally, the first sensor is located at the first fixed location and the second sensor is located at the second fixed location.

Optionally, the degree of tilt is determined based on a ratio between the first distance and the second distance.

Optionally, the patient positioning apparatus has a major axis and a minor axis, and the first and second fixed locations are separated along the major axis when the patient positioning apparatus is at zero tilt, such that the processor is configured to determine, based on signals from the sensors, the degree of pitch of the patient support apparatus.

Optionally, the patient positioning apparatus has a major axis and a minor axis, and the first and second fixed locations are separated along the minor axis when the patient positioning apparatus is at zero tilt, such that the processor is configured to determine, based on signals from the sensors, the degree of roll of the patient support apparatus.

Optionally, the apparatus further comprises a third sensor being configured to provide signals indicative of a third distance between a third region of the underside of the patient support apparatus and a third fixed location underneath the patient support apparatus, the first, second and third sensors being positioned to allow the processor to determine, based on signals from the sensors, the degree of both pitch and roll of the patient support apparatus.

Optionally, the processor is further configured to determine, based on signals from the first, second and third sensors, the height of a fourth region of the underside of the patient support apparatus; wherein the fourth region lies on a plane defined by the first, second and third regions.

Optionally, the patient positioning apparatus is configured to tilt about a pitch rotation axis and tilt about a roll rotation axis.

Optionally, wherein the major axis when the patient positioning apparatus is at zero tilt is parallel with and/or aligns with the roll axis.

Optionally, wherein the minor axis when the patient positioning apparatus is at zero tilt is parallel with and/or aligns with the pitch axis.

Optionally, wherein the patient support apparatus comprises a patient support base structure and a patient support surface; the patient support surface tiltable about a roll rotation axis with respect to the base structure; and wherein the apparatus comprises a roll rotation sensor coupled to the processor and configured to provide signals indicative of a distance between a first region of the underside of the patient support surface and a fixed location between the patient support base structure and the patient support surface.

Optionally, wherein the processor is configured to determine a degree of pitch of the patient support apparatus based on signals received from the first and second sensors, and a degree of roll based on signals received from the roll rotation sensor.

Optionally, wherein the processor is configured to determine the height of any point of the patient support apparatus along a line joining the first and second regions of the underside of the patient support apparatus based on signals received from the first and second sensors.

Optionally, wherein the apparatus comprises a pitch rotation mechanism configured to tilt the patient support apparatus about a pitch rotation axis.

Optionally, wherein the sensors are configured to measure distances along a direct line of sight.

Optionally, wherein one or more sensors of the sensor arrangement comprise one of an optical sensor, a draw wire sensor, or a linear travel sensor.

In particular disclosed arrangements, a method of determining a degree of tilt of the patient support apparatus according to any preceding item comprises determining the degree of tilt of the patient support apparatus based on signals received from the sensors.

In particular disclosed arrangements, the method comprises determining a first value based on signals received from the first sensor, determining a second value based on signals received from the second sensor, and determining the degree of tilt based on a ratio of the first and second value.

Optionally, the patient support apparatus comprises a third sensor configured to provide signals indicative of a third distance between a third region of the underside of the patient support apparatus and a third fixed location underneath the patient support apparatus; the method further comprising determining, based on signals from the sensors, the degree of both pitch and roll of the patient support apparatus.

Optionally, the method further comprising determining a third value based on signals received from the third sensor; determining the degree of pitch of the patient support apparatus based on the ratio of the first and second value; and determining the degree of roll of the patient support apparatus based on the ratio of the second and third value.

Optionally, the method further comprising determining, based on signals from the first, second and third sensors, the height of a fourth region of the underside of the patient support apparatus; wherein the fourth region lies on a plane defined by the first, second and third regions.

Optionally, wherein the patient support apparatus comprises a patient support base structure and a patient support surface; the patient support surface tiltable about a roll rotation axis with respect to the base structure; and wherein the apparatus comprises a roll rotation sensor coupled to the processor and configured to provide signals indicative of a distance between a first region of the underside of the patient support surface and a fixed location between the patient support base structure and the patient support surface; the method comprising determining a degree of pitch of the patient support apparatus based on signals received from the first and second sensors, and a degree of roll based on signals received from the roll rotation sensor.

In particular disclosed arrangements, a computer-readable medium comprises computer executable instructions which, when performed by a computer, cause the computer to carry out the methods disclosed herein.

### A patient support apparatus for tilting a patient support surface with respect to a patient support base

The present application also discloses a patient support apparatus comprising a patient support surface which is tiltable with respect to a patient support base structure. This patient support apparatus may be used in combination with, or separately from, the other arrangements disclosed in this application.

One implementation of such a patient support apparatus comprises a tiltable patient support surface that is configured to be tilted with respect to the patient support apparatus. The patient support surface is configured to be rotated about an axis of rotation referred to herein as a roll axis or a tilt axis. Thus, the patient support surface may be described as a tiltable patient support surface. In this example, the axis of rotation is parallel to the longitudinal axis of the patient support surface (roll axis 1120, as shown in figure 10). In other examples, the axis of rotation may instead be parallel to the lateral axis of the patient support surface (pitch axis 1110, as shown in figure 10).

Disclosed herein is a patient support apparatus comprising a tiltable patient support surface. The apparatus comprises a base structure which comprises a first and a second drive shaft, and one or more rotary motors configured to rotate the first and second drive shafts. The patient support surface comprises a first and a second coupling member, and the coupling members extend from a lower surface of the patient support surface. The first coupling member is rotationally coupled to the first drive shaft via a first swing element and the second coupling member is rotationally coupled to the second drive shaft via a second swing element. Each swing element extends radially outward from its drive shaft and is configured to rotate with the drive shaft. This structure means that, by rotating each of the first and second drive shafts in a first direction, the patient support surface is caused to tilt in the first direction with respect to the base structure, and by rotating each of the first and second drive shafts in a second direction, the patient support surface is caused to tilt in the second direction with respect to the base structure.

For example, arrangements of the present disclosure may be implemented with the radiotherapy device 100 shown in figure 1. Figure 1 shows an example of a non-coplanar radiotherapy device that combines the rotation of the patient with the rotation of the radiation source. In the present arrangement, the patient support apparatus 114 is configured to be rotatable around the vertical z-axis, while the gantry 116 is configured to be rotatable about the longitudinal y- axis around the patient support apparatus 114. The gantry 116 is a C-arm gantry or open gantry. A rotation mechanism rotates the gantry 116 about the y-axis. As the gantry 116 is rotated, radiation is emitted by a radiation source 106 along a radiation axis and around a circle that lies in a radiation plane. Radiation is therefore delivered to the patient from a plurality of angles in the radiation plane.

In the example of figure 1, a rotation mechanism, disposed under the gantry 116 is provided to rotate the patient support apparatus 114 about an axis of rotation of the patient support apparatus 114 in the radiation plane that coincides with the z-axis. In particular, the axis of rotation of the patient support apparatus coincides with the isocenter of the radiotherapy device, such that the patient support apparatus rotates about the isocenter. A rotation mechanism is provided for the gantry 116 disposed opposite the patient support apparatus 114 with respect to the axis of rotation. In the following discussion, the vertical z-axis is taken to be the axis perpendicular to the plane of the patient support apparatus when it is in its neutral position (parallel to the plane of the floor), the transverse x-axis is taken to be the transverse axis (short side) of the patient support apparatus, and the longitudinal y-axis is taken to be the longitudinal axis (long side) of the patient support apparatus. In the example of figure 1, the axis of rotation of the patient support surface 114 coincides with the vertical z-axis.

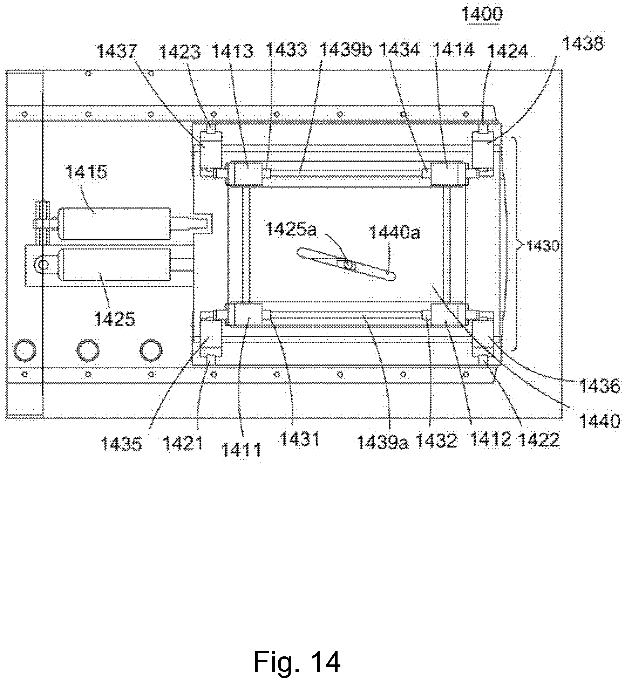

Figures. 13 and 14 show an exemplary patient support apparatus 1400 in accordance with an arrangement. The patient support apparatus 1400 may be implemented in the patient support apparatus 114 of the radiotherapy device 100 and/or as part of any of the patient positioning apparatus(es) disclosed herein, such as the patient positioning apparatus of figures 2a-12. The patient support apparatus 1400 comprises a patient support 1410, and a base 1420. The patient support 1410 may be referred to as a patient support surface. During radiotherapy, a subject (e.g. a human patient) is positioned on, and supported by, the top surface of the patient support 1410. The base 1420 supports the patient support 1410. The patient support 1410 and the base 1420 define a space therebetween and are parallel with each other when the patient support surface 1410 is in a neutral position.

In the arrangement, the patient support apparatus 1400 further comprises first guides 1421, 1422, 1423, 1424 disposed on the base 1420, a plate 1440 with an angled elongated groove or slit 1440a, a first actuator 1425 disposed within a groove in the base 1420 with an extendable end having a protrusion 1425a movably coupled to the elongated groove 1440a of the plate 1440 (see figure 14), and first guide blocks 1435, 1436, 1437, 1438 disposed at the four corners of the plate 1440 configured to slide along the inclined surface of the corresponding first guides 1421, 1422, 1423, 1424. The elongated groove 1440a is provided in the plate 1440 along a groove axis that is at an angle relative to the longitudinal y-axis. In an arrangement, the elongated groove 1440a is at an angle in the range of 20 degrees to 30 degrees relative to the longitudinal y-axis. The first actuator 1425 and the plate 1440 may collectively be referred to as a tilting module 1430. In the present arrangement, four first guides 1421, 1422, 1423, 1424 and four first guide blocks 1435, 1436, 1437, 1438 are provided proximate to the four corners of the plate 1440. Providing the first guides and first guide blocks proximate to the edge of the plate 1440, and in particular proximate to the corners of the plate 1440, provides a wide base that gives stability to the structure, and allows the weight of the patient support 1410 to be distributed more uniformly compared to supporting and rotating the patient support on a single pivot point. However, more or fewer first guides and corresponding first guide blocks may be used as desired, and they may be disposed at different locations relative to the plate 1440 alternatively or in addition to the corners. For example, only two first guides may be provided with one along each of the long side of the plate 1440, or six first guides may be provided with four arranged as shown in the arrangement of figure 13 and an additional guide along each of the long side of the plate 1440. Preferably each of the guide is provided with a corresponding guide block. In preferred arrangements, the angle of inclination between the inclined surface of each of the first guides relative to the base 1420 is within a range of 20 degrees to 40 degrees. Preferably the angle of inclination is within a range of 25 degrees to 35 degrees. Most preferably the angle of inclination is at approximately 30 degrees.

In operation, when the first actuator 1425 extends, the protrusion 1425a at the extendable end of the first actuator 1425 travels along the elongated groove 1440a. Since the first actuator 1425 is fixed in its position relative to the base 1420, as the ball bearing 1425a moves along the elongated groove 1440a, the plate 1440 is pushed or shifted in the transverse x-direction. In turn, the movement of the plate 1440 causes the first guide blocks 1435, 1436, 1437, 1438 to slide along the inclined surfaces of the first guides 1421, 1422, 1423, 1424. In the present arrangement, as the first guide blocks 1435, 1436, 1437, 1438 slide along the corresponding first guides 1421, 1422, 1423, 1424 as a result of the plate 1440 being moved along the x-axis relative to the base 1420, the plate 1440 is lifted relative to the base on a side where two guide blocks slide up the corresponding guides and is lowered on the opposite side where two guide blocks slide down the corresponding guides such that the plate 1440 is tilted about the longitudinal y-axis relative to the base 1430 and no longer parallel to the base 1430. In the present arrangement, the first guide blocks 1435, 1436, 1437, 1438 are rotatably coupled to the plate 1440 is rotatable at each of the long side of the plate 1440, such that the sliding of the first guide blocks 1435, 1436, 1437, 1438 along the inclined first guides 1421, 1422, 1423, 1424 result in a gradual tilt of the plate 1440. In the present arrangement, the patient support 1410 is coupled to the plate 1440 in such a way that the patient support 1410 moves parallel to the plate 1440, such that the tilting of the plate 1440 causes corresponding tilting in the patient support 1410 about the longitudinal y-axis (roll). Effectively, the plate 1440 is configured, through the angled elongated groove 1440a, to translate the movement generated by the first actuator 1425 that is along the longitudinal y-axis, into the movement of the patient support 1410 along the first guides 1421, 1422, 1423, 1424 that are inclined along the transverse x-axis. While the present arrangement shows an arrangement in which the first actuator 1425 generates movement in the y-direction that is translated by the plate 1440 into movement in the x-direction, alternative arrangement may be used in which the first actuator 1425 may be provided to generates movement in the x-direction, e.g. by rotating the first actuator 1425 by 90 degrees, that is translated by the plate 1440 into movement in the y-direction to cause tilting in the plate, therefore the patient support 1410, about the x-axis (pitch). Although in the present arrangement an actuator (first actuator 1425) is used to generate linear movement, a different drive unit may be user if desired.

In the present arrangement, the patient support apparatus 1400 may optionally further comprise second guides 1431, 1432, 1433, 1434 disposed inwards from the four corners of the plate 1440, and second guide blocks 1411, 1412, 1413, 1414 disposed on the underside of the patient support 1410. A second actuator 1415 is rotatably coupled to the underside of the patient support 1410 at one end and rotatably attached to the plate 1440 at an extendable end to enable vertical movement of the patient support 1410 allowing it to be lifted or lowered relative to the base 1420 by the motion of the second actuator 1415. The plate 1440 has two cut-out sections 1439a, 1439b at opposing sides of the plate 1440 along the longitudinal y-axis, which allow the second guide blocks 1411, 1412, 1413, 31414 to slide up and down the inclined surface of the second guides 1431, 1432, 1433, 1434. In the present arrangement, four second guides 1431, 1432, 1433, 1434 and four second guide blocks 1411, 1412, 1413, 1414 are provided at the four corners of the plate 1440 inward from the first guides and first guide blocks. However, more or fewer second guides and corresponding second guide blocks may be used as desired, and they may be disposed at different locations relative to the plate 1440 alternatively or in addition to the corners. In preferred arrangements, the angle of inclination between the inclined surface of each of the second guides relative to the base 1420 is within a range of 20 degrees to 40 degrees. Preferably the angle of inclination is within a range of 25 degrees to 35 degrees. Most preferably the angle of inclination is at approximately 30 degrees.

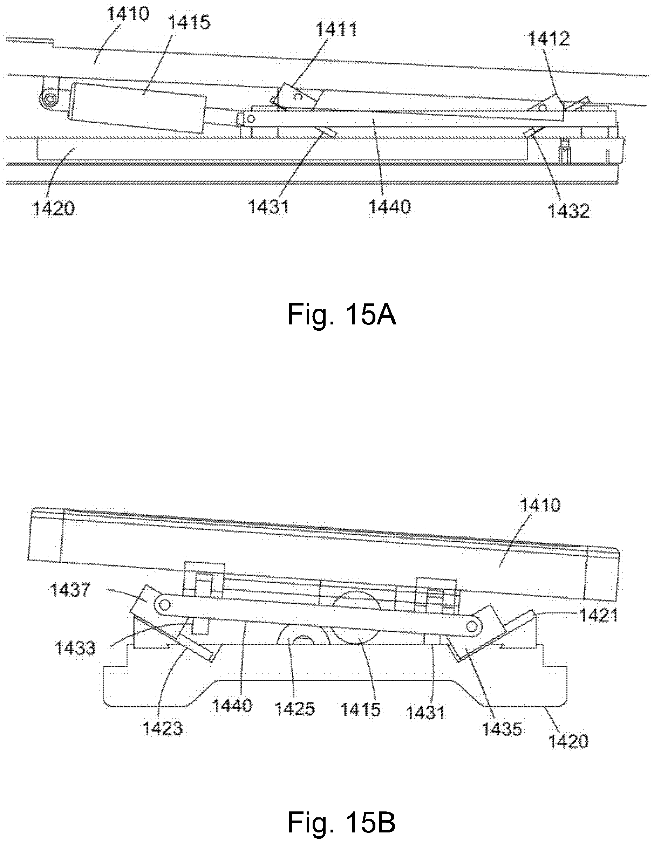

As can be seen in figures 15a and 15b, in the present arrangement, the first guides 1421, 1422, 1423, 1424 are arranged to inclined away from the central longitudinal axis of the base 1420, while the second guides 1431, 1432, 1433, 1434 are arranged to inclined away from the central transverse axis of the plate 1440. Such arrangement has an advantage of biasing the patient support 1410 towards a central neutral position when the patient support 1410 is parallel to the base 1420 (horizontal). Moreover, providing the first guides, and optionally the second guides, along opposing sides of the plate 1440, and preferably proximate to the corners of the plate 1440 according to the present arrangement, allows a subject supported by the patient support 1410 to remain at the same height with respect to the ground, such that the potential energy of the subject remains unchanged irrespective of the amount of tilting around the x-axis (pitch) and/or the y-axis (roll). In other words, the centre of gravity of the subject remains at the same height from the ground. Since the weight of the subject is not lifted or lowered whether the patient support is pitched or rolled, the amount of energy input required to pitch or roll the patient support is significantly lower, which allows relatively small motors, drive units or actuators to be used, leading to a cheaper more compact patient support apparatus.

In operation, the second actuator 1415 extends to push against the plate 1440, causing the patient support 1410 to move in the longitudinal y-direction, which in turn causes the second guide blocks 1411, 1412, 1413, 1414 to move along the inclined surfaces of the second guides 1431, 1432, 1433, 1434. As such, the patient support 1410 is lifted relative to the base on a side where two guide blocks slide up the corresponding guides and is lowered on the opposite side where two guide blocks slide down the corresponding guides, such that the patient support 1410 is tilted about the transverse x-axis relative to the base 1430 (pitch). Although in the present arrangement an actuator (second actuator 1415) is used to generate linear movement, a different drive unit may be user if desired.

As can be seen in the present arrangement, the configuration of the plate 1440 enables the first actuator 1425 to be positioned adjacent the second actuator 1415, allowing the overall arrangement of the tilting module 1430 to be compact. The use of an angled elongated groove 1440a in the plate 1440 represents an example of how the plate 1440 may be configured to achieve such translation of movement from one direction to a perpendicular direction; however, other suitable implementations may be possible. In the present arrangement, the plate 1440 is configured to translate the movement of the first actuator 1425 along the longitudinal y-axis into the movement of the patient support 1410 along the transverse x-direction. However, it has been contemplated that the first actuator 1425 may alternatively be arranged to generate movement in the transverse x-direction, and the plate 1440 may then be configured to translate the movement of the first actuator 1425 along the transverse x-axis into the movement of the patient support 1410 along the longitudinal y-direction, if desired.

In some arrangements, the operation of the first and second actuators 1425, 1415 may be controlled by means of a software program executed by a processor. However, the operation of the first and second actuators 1425, 1415 may be manually controlled if desired.

According to arrangements of the present disclosure, the patient support of the patient support apparatus 114 may be tilted or rotated about the transverse (x-) axis (pitch) and/or tilted or rotated about the longitudinal (y-) axis (roll). Thus, according to the present disclosure, it is possible to position a patient with an additional two degrees of freedom. The additional two degrees of freedom (pitch and roll) may be adjusted independently or in combination, and whilst the couch is in a neutral rotational position (yaw) or when it is in a rotated position. For patient's safety and comfort, the amount of pitch and roll is preferably limited to a predetermined maximum angle. Arrangements of the present disclosure provides an arrangement using simple mechanical elements to achieve one or more additional degrees of freedom in pitch and/or roll, the movement of which can be controlled straightforwardly due to the independent and predictable movement of the support surface in the transverse or longitudinal directions along the inclined surfaces of the guides. The simplicity of the arrangement allows the size of the couch to be kept relatively compact, enabling its centre of gravity to be kept low. The rotation of the support surface about the transverse and longitudinal axes can be arranged to pivot around the same point at the centre of the plate, and as such any compensations required in the x-, y- or z-direction may be minimised. The disclosed arrangement utilising opposing guides limits or prevents the movement of the plate 1440, and therefore the patient support 1410, to the direction of the axes of the first, and optionally second, guides. In other words, rotation around the z-axis is restricted, or locked, by the arrangement of the guides according to preferred arrangements. As such, additional stabilizing mechanism is not required to prevent accidental rotation around the z-axis while the support surface 1410 is rolled or pitched. The positions of the guides provide a wide base for receiving the weight of the support surface and the subject thereon, thereby improving the stability of the support surface. In some examples, the couch 114 or section thereof may be pitched or rolled about an axis that is spaced apart from the isocenter whilst a different section may be moved to compensate and maintain a portion of the couch 114 substantially at the isocenter. In this way, it is possible to maximise the spread of the radiation through the healthy tissue whilst maximising the dose of radiation that is delivered to the target region.

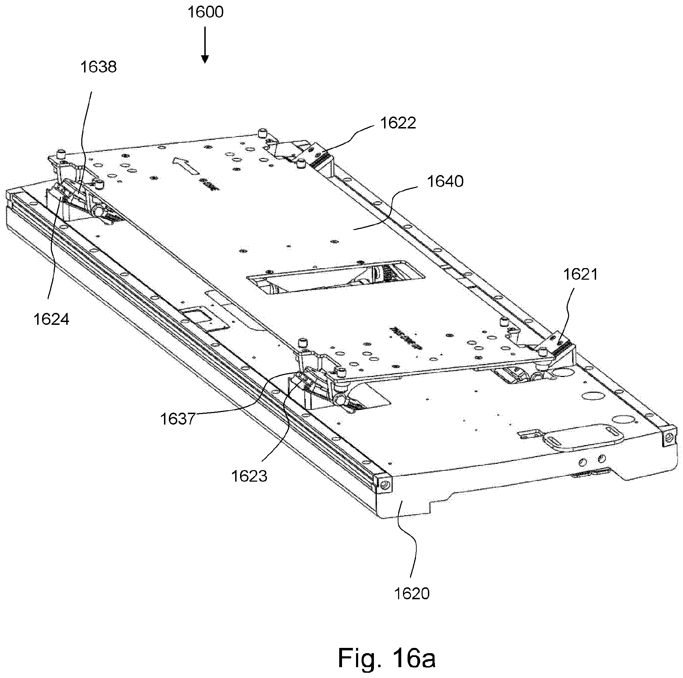

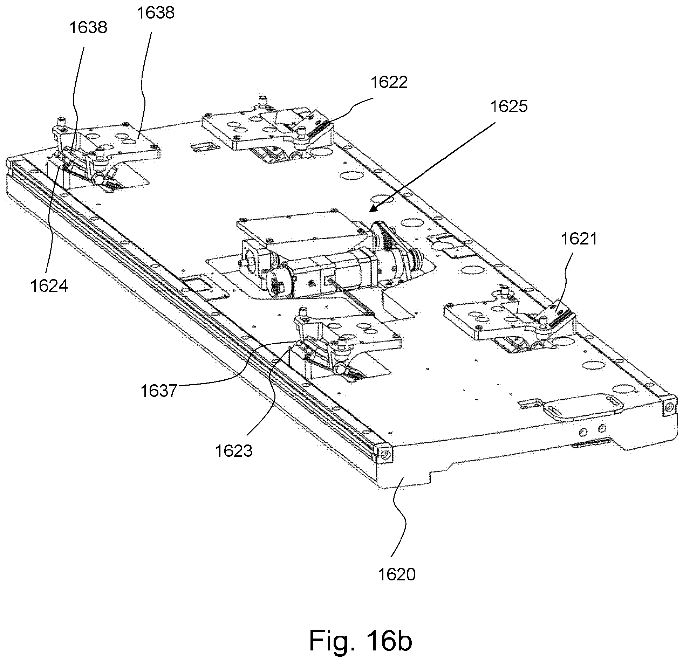

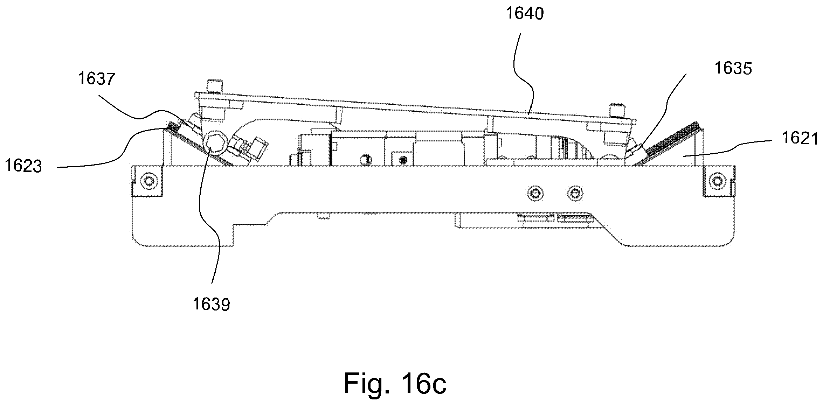

Figures 16a, 16b, and 16c depict an arrangement according to the present disclosure. The description above, which describes the example arrangements depicted in figures 13-15, is broadly applicable to the present arrangement except where otherwise noted below, and like features are depicted using like reference numerals where appropriate to facilitate understanding.

Figures 16a-c depict an exemplary patient support apparatus 1600, though, to better depict the coupling of plate 1640 to base 1620, the apparatus 1600 is depicted without a patient support. In a fully assembled patient support apparatus 1600, the base 1620 supports a patient support such that a subject (such as a patient) may be positioned on and supported by the patient support in a manner similar or identical to that described above in relation to figures 13 to 15. Figure 16a is an angled view depicting a patient support apparatus 1600 comprising a base 1620 coupled to a plate 1640. Figure 16b depicts the same angled view of the patient support apparatus 1600 but with the plate 1640 removed so as to better depict the arrangement of a first actuator 1625. Figure 16c depicts a front view of the same patient support apparatus 1600 depicted in figure 16a.

When fully assembled with a patient support coupled to the plate 1640, the patient support is tiltable with respect to the base 1640. The patient support may be tiltable about a first rotation axis, which may be a fixed rotation axis. The first rotation axis is parallel with and/or may be coincident with the longitudinal centre line of the patient support and/or the longitudinal centre line of the plate 1640 and/or the longitudinal centre line of the base 1620.

In the depicted arrangement, the patient support apparatus 1600 further comprises first guides 1621, 1622, 1623, 1624 disposed on the base 1620. The first guides 1621, 1622, 1623, 1624 are inclined with respect to the base 1620. The first guides 1621, 1622, 1623, 1624 are fixed with respect to the base 1620 and extend into recesses in the base 1620. By providing inclined guides 1621, 1622, 1623, 1624 which extend into recesses in the base, a gap between the base 1620 and a patient support surface can be advantageously reduced for a given degree of available tilt. Patients undergoing radiotherapy may be frail and their mobility may be limited, and reducing the 'hop-on' height of a patient support apparatus, i.e. the height of the patient support above the floor when in a neutral tilt position, is advantageous as it allows patients to more easily mount and position themselves on the patient support. Reducing the gap between the base 1620 and the patient support is also advantageous for other reasons, for example because it reduces the chances that something may be caught in this gap, for example the fingers of a patient or clinician, as the patient support tilts with respect to the base 1620.

As can be appreciated from the figures, the first guides 1621, 1622, 1623, 1624 are arranged to incline away from a centre of the plate 1640. More specifically, the first guides 1621, 1622, 1623, 1624 are arranged to incline away from a centre line of the plate 1640. In the arrangement depicted, the first guides 1621, 1622, 1623, 1624 incline away from a longitudinal centre line of the plate 1640 and from the first rotation axis. A first subset of the first guides, denoted by reference numerals 1621 and 1622, are positioned on a first side of the base 1620, and a second subset of the first guides, denoted by reference numerals 1623 and 1624, are positioned on an opposing, second side of the base 1620. The first and second subset of the first guides are thereby positioned either side of the first rotation axis. The guides 1621, 1622 of the first subset of guides incline away from the centre of the plate and/or away from the first rotation axis in a first direction, and the guides 1623, 1624 of the second of guides incline away from the centre of the plate and/or away from the first rotation axis in a second direction, with the first direction being opposite the second direction. Each of the first subset of guides 1621, 1622 faces a respective one of the second subset of guides 1623, 1624. Accordingly, the patient support is biased to move towards a central neutral position, thus improving the stability of the patient support and guarding against large and sudden changes in the patient support tilt angle should there be a mechanical failure of any kind.

The apparatus 1600 further comprises first guide blocks 1635, 1636, 1637, 1638 disposed on the plate 1640. In the arrangement depicted in the figures, the first guide blocks 1635, 1636, 1637, 1638 are disposed at the four corners of the plate 1640. As with the arrangement described with respect to figures 13 to 15, the first guide blocks 1635, 1636, 1637, 1638 are configured to slide along the inclined surface of the corresponding first guides 1621, 1622, 1623, 1624. The guides 1621, 1622, 1623, 1624 may comprise a plurality of guide rails in the manner described above, and the guide blocks 1635, 1636, 1637, 1638 may comprise a plurality of corresponding first carriages arranged to slide along the plurality of guide rails. The plurality of first carriages may be rotatably coupled to the plate 1640 via a rotational coupling. For example, the rotational coupling 1639 depicted in figure 16c couples plate 1640 to a carriage which forms part of guide block 1637. These rotational couplings allow the plate 1640 to tilt as the carriages move up and down the inclined surfaces of the guide rails.

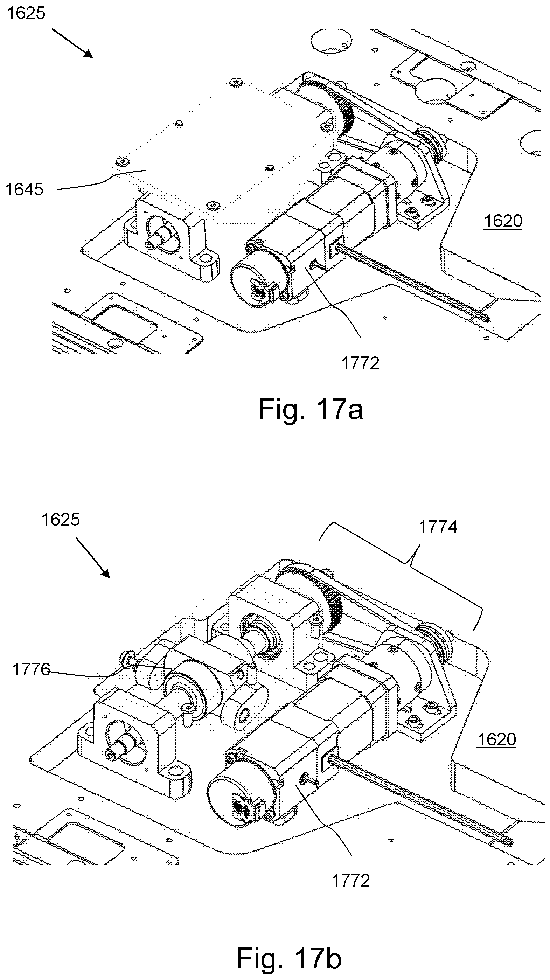

As with other arrangements described herein, the apparatus 1600 comprises a tilting module positioned between the base 1620 and the patient support which comprises the plate 1640 and a first actuator 1625. The first actuator 1625 can best be inspected in figures 17a and 17b. The plate 1640 is coupled to an underside of the patient support such that the patient support tilts with the plate 1640. The plate 1640 is also coupled to the first actuator 1625 such that movement generated by the first actuator 1625 is translated into movement of the plate 1640 along the first guides 1621, 1622, 1623, 1624, thereby causing the patient support to tilt relative to the base 1640.

The first actuator 1625 is disposed on the base 1620. The first actuator is positioned in a recess of the base 1620, again so as to reduce the 'hop-on height', i.e. the gap between base 1620 and the support when the apparatus 1600 is in a neutral tilt position. The first actuator 1625 is a linear actuator and comprises an electric motor 1772, a belt and pulley arrangement 1774, and a lead screw or ball screw 1776. The skilled person will appreciate that the first actuator 1625 may take different forms in order to achieve the functionality described herein, and that rather than the form depicted in figures 17a-17b the first actuator 1625 may take any of a plurality of forms; for example, a simple piston as depicted in figures 13-15.

The plate 1640 is coupled to the first actuator 1625 via a motion converter 1645, which may be referred to as a motion converting unit or a motion converting element. The motion converter 1645 may have a triangular prism shape. The motion converter 1645 is rotationally coupled to the first actuator 1625. The motion converter 1645 comprises axles or spindles which are configured to cooperate with apertures or indentations on the first actuator 1625 in order to define a rotational coupling between the motion converter 1645 and the linear actuator 1625. Of course, the provision of spindles and corresponding indentations or apertures may be reversed. The motion converter 1645 is rigidly coupled to the underside of the plate 1640 and may form part of the plate 1640.

In operation, when the first actuator 1625 extends, the motion is passed via the motion converter 1645 to the plate 1640. Motion of the first actuator 1645 causes movement of the motion converter 1645, which in turn causes a tilting action of the plate 1640, which in turn causes the patient support to tilt. In a manner similar to the second actuator 1415 described above and depicted in figure 15a, motion of the first actuator 1625 along a movement axis is translated to, i.e. causes, movement of the plate 1640 along the inclined first guides 1621, 1622, 1623, 1624, thereby causing the patient support to tilt about a first rotation axis relative to the base. In the arrangement depicted in figures 16a-c, 17a and 17b, this rotation axis is substantially perpendicular to the movement axis of the first actuator 1645.

By coupling the first actuator 1625 to the plate 1640, e.g. via the motion converter 1645, force is applied directly to the tiltable plate. Because the tiltable plate is coupled directly to the tiltable patient support, an efficient transfer of energy and force is provided from the actuator, to the plate, and to the tiltable patient support. This is advantageous over a design which seeks to instead effect a tilting action by applying a force to a moveable inclined surface underneath the patient support in order to force the patient support upward, thereby causing a tilting action about a pivot point. Such a design would require the pivot point to be anchored between the base and upper surface, and such a design would create unnecessary strains and stresses at the pivot point and thus would be more susceptible to mechanical failure.

The patient support apparatus 1600 is depicted in the figures as having an axis of rotation parallel to the longitudinal axis of the patient support. This may be described as a roll rotation in the art.

However, it should be appreciated that the same mechanism may be used to provide a pitch rotation via reconfiguration and reorientation of the first guides. The patient support apparatus 1600 is depicted in the figures as being tiltable about only one axis of rotation, however it should be appreciated that a plurality of second inclined guides and second guide blocks may be provided, in the manner described herein, so as to allow the patient support of apparatus 1600 to tilt about a second axis of rotation.

Disclosed herein is a patient positioning apparatus for a medical device, the apparatus comprising a patient support apparatus, a support structure configured to extend between the patient support apparatus and a floor of a treatment room to support the patient support apparatus above the floor of the treatment room, wherein the patient support apparatus is rotationally coupled to the support structure; and a rotation mechanism comprising a drive member and configured to impart a force, via the drive member, to an underside of the patient support apparatus to thereby rotate the patient support apparatus with respect to the support structure; wherein the rotation mechanism is attached to, and supported by, the support structure.

Optionally, wherein the rotation mechanism is rotationally coupled to the support structure.

Further optionally, wherein the drive member of the rotation mechanism is rotationally coupled to the support structure.

Further optionally, wherein the patent support apparatus comprises a patient support surface and a patient support base, the patient support surface being configured to translate linearly with respect to the patient support base, and wherein the support structure is rotationally coupled to the patient support base.

Further optionally, wherein the support structure comprises a base configured to contact and/or be embedded in the floor of the treatment room.

Further optionally, wherein the rotation mechanism is positioned between the patent support apparatus and the base.

Further optionally, wherein the support structure comprises a height adjustment mechanism configured to control a height of the patient support apparatus above the floor of the treatment room.

Further optionally, wherein the patient support apparatus is rotationally coupled to the support structure to allow rotation about a principal rotation axis, and wherein controlling the height of the patient support apparatus comprises controlling the height of the principal rotation axis above the floor of the treatment room.

Further optionally, wherein the height of the patient support apparatus and the rotation of the patient support apparatus are controllable independently of one another.

Further optionally, the support structure further comprising a support element and a supporting leg, wherein the supporting leg is rotationally coupled to both the patient support apparatus and the support element, and wherein the height adjustment mechanism comprises a motor mechanism configured to rotate the supporting leg with respect to the support element and thereby control the height of the patient support apparatus.

Further optionally, wherein the rotation mechanism is attached to the support element.

Further optionally, wherein the rotation mechanism comprises an actuation mechanism configured to control movement of the drive member.

Further optionally, wherein the drive member comprises an aperture and is rotationally coupled to the support structure via an axle which extends through the aperture; and wherein the actuation mechanism further comprises a motion converter which extends through the aperture and is eccentrically mounted with respect to the axle such that rotation of the motion converter about the axle causes linear movement of the drive member.

Further optionally, wherein the actuation mechanism comprises a linear actuator coupled to a crank arm of the motion converter such that actuation of the linear actuator causes the motion converter to rotate about the axle.

Further optionally, wherein the drive member is coupled to the underside of the patient support apparatus via a coupling element; the drive member being rotationally coupled to the coupling element at a first coupling point and rotationally coupled to the support structure at a second coupling point; and wherein the actuation mechanism is configured to move the drive member by adjusting a distance between the first and the second coupling point.

Each of the motors, actuators, and other mechanisms configured to effect movement described herein are controllable by one or more processors. In particular, the height adjustment mechanism and the rotation mechanism are controllable by one or more processors such that these mechanisms can be controlled both independently of one another, and/or together, depending on the requirements of the medical procedure, calibration process, etc..

The approaches described herein, for example positioning control instructions to control the rotation and height adjustment mechanisms, may be embodied on a computer-readable medium, which may be a non-transitory computer-readable medium. The computer-readable medium carrying computer-readable instructions arranged for execution upon a processor so as to make the processor carry out any or all of the methods described herein.

### Accuracy characterisation and control

Disclosed herein is a method and system for estimating, or characterising, the positioning accuracy, or error, of a patient positioning system. Also disclosed herein is a method and system for controlling the patient positioning system in order to improve the patient positioning accuracy, i.e. to reduce a discrepancy between an estimated position and a reference position, for example to achieve a particular level of positioning accuracy. The patient positioning system may comprise any of the patient support apparatus(es) and mechanisms disclosed herein, such as those of figures 1-17b. The accuracy with which the overall patient positioning system can reproducibly position the target tissue of the patient at the radiation delivery isocentre is important to ensuring that the prescribed dose of radiation is delivered accurately and optimally to the target region.

Various offsets, discrepancies, shifts, and/or deviations in position can affect the accuracy with which the patient positioning system can be moved, translated, rotated, and/or positioned. As used herein, the terms "error", "offset", "discrepancy", "shift", "additional motion", and "deviation" are considered equivalent and are used interchangeably, and are considered to mean a difference between an expected, intended, or idealised position of a point or region in three-dimensional space and the actual, or achieved, position of the point or region in three-dimensional space. In particular, the term "offset" is used herein with the intention of referring to a discrepancy, rather than the alternative meaning of the word "offset" as referring to a factor to be applied in order to correct such a discrepancy. Such a factor will instead be referred to herein using variations of the terms "compensate" or "compensation", or may be referred to as a "correction" or "corrected".

A number of factors may produce discrepancies between the idealised, or expected, position of a point on the patient positioning system or region of the patient and the actual position achieved by the system. Mechanical deformation, bending, and/or sag of patient support apparatus components can all produce a discrepancy between a desired position of the patient and the actual position of the patient. To complicate matters further, the degree of deformation, bending, and/or sagging often depends on factors associated with the patient, such as the patient's weight and the distribution of that weight.

The position of the patient support apparatus and/or patient support surface may deviate from the intended position in one or more of the X, Y, and Z coordinates described herein. The position of the patient may hence also deviate accordingly. A deviation in one or more coordinates may be caused by offsets inherent in, or produced by, the mechanisms used to rotate the patient support surface, such as due to backlash from a component part. A deviation in one or more coordinates may alternatively or additionally be caused by mechanical bending or deformation of components of the patient positioning system. Such bending or deformation may be inherent to the system or the mechanisms used by the system or may be produced or exacerbated by the presence of a patient on the patient support surface.

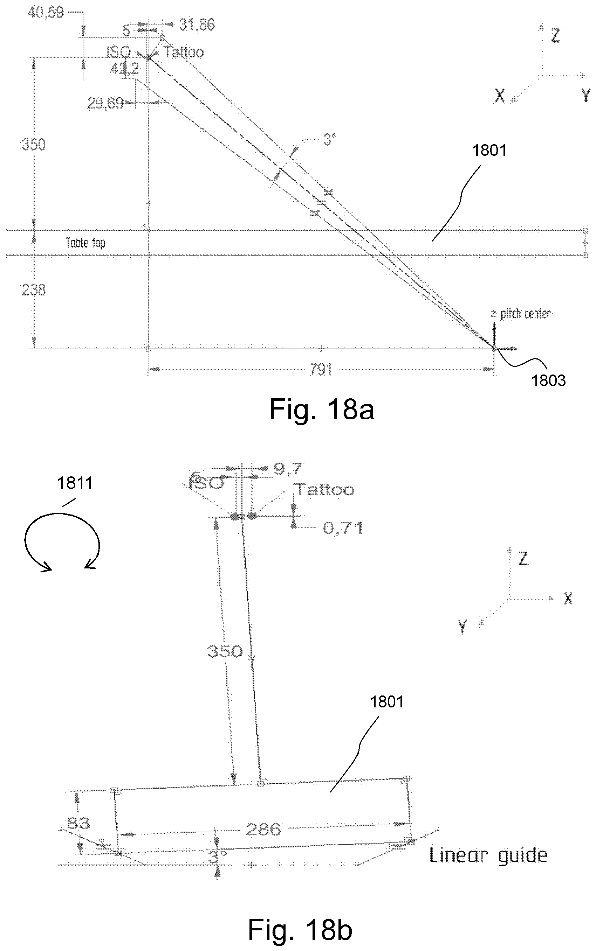

Figure 18a shows an example of a position deviation that can occur during pitch rotation of a patient support surface 1801, such as a rotation by the pitch rotation mechanism of any of figures 2a-12. With a patient support apparatus of particular dimensions and a particular maximum range of pitch angle, a particular position discrepancy in each of the Y and Z directions is produced.

The patient support surface 1801 may be in accordance with any of the examples described above, and has a particular length, width, and thickness. In the example of figure 18a, a top surface of the patient support surface 1801 is positioned 238 mm in the Z direction above a central point 1803 around which the pitch rotation of the patient support surface 1801 is configured to rotate. A patient is provided with a tattoo, which may be a temporary mark on the patient's skin that marks the intended entry point of irradiation into the patient. In the present example, the patient is positioned on the patient support surface 1801 such that the tattoo is located 350 mm above the top surface of the table top and is located 791 mm in the Y direction from the centre of pitch rotation. It will be appreciated that the location, or height, of the tattoo above the top surface of the table top will vary according to the size of the patient and the position of the tattoo on the patient, and that 350 mm is merely chosen as an example value of this height. In other examples, a different height may be used and the geometry may be adjusted accordingly such that different system and/or patient dimensions are taken into account to estimate the error values and accuracy of that particular arrangement.

As indicated using exemplary dimensions in figure 18a, when rotated to the end points of its rotation range, for example ±3°, the pitch rotation mechanisms described herein can produce additional, sub-optimal translation motion. With the exemplary dimensions indicated on figure 18a, the difference between the point labelled "ISO", which refers to the position of radiation delivery of the system, and the point labelled "tattoo", which refers to the target location on the skin of the patient, can be up to 32 mm in the Y direction and 42 mm in the Z direction. For treatment to be optimised, these points should align with one another, so these errors need to be compensated for. In a method according to the present disclosure, these errors may be described as compensation values. Having rotated the PPS in a pitch direction, translation errors are introduced, which can be corrected for, or at least mitigated, by linearly adjusting the PPS by these compensation values using the translational movement mechanisms. Therefore, in order to compensate for the position discrepancy introduced when the systems disclosed undergo a pitch rotation movement, an additional compensation movement in the Y and/or Z direction may be implemented to counteract the characterised position discrepancies. Thus, patient positioning accuracy is improved. Although pitch rotation motion according to the mechanisms described herein typically introduces additional motion in the Y and Z directions, in other implementations a pitch rotation movement may introduce additional motion in any one or more of the Y, Z, and X coordinates, which may be compensated accordingly. Likewise, although in the examples of this disclosure the discrepancy is characterised as a difference between the point of radiation delivery (ISO) and the tattoo on the patient's skin, in other examples the position discrepancy may be characterised as a difference between the isocentre of the radiation delivery and an intended point of irradiation within the patient, such as within a tumour.

Figure 18b is a simple schematic showing the patient support surface 1801 of figure 18a from another perspective. By using a roll rotation mechanism, the patient support surface 1801 can be rotated around a roll axis in either of the directions shown by the curved double-headed arrow 1811. Figure 18b shows a discrepancy which may be introduced by a roll mechanism such as that of figure 16c. While figure 18b depicts the linear guides described with respect to figure 16c, the present method is compatible with any of the roll motion mechanisms disclosed herein (such as any of those of figures 13-17). When the roll mechanism is rotated to a maximum of ±3°, additional unintended motion in the X and Z directions may be produced of up to 10 mm and 1 mm respectively. Like in figure 18a, the additional motion of figure 18b produces a discrepancy between the point of radiation delivery, labelled "ISO", and the intended target on the patient's skin, labelled "tattoo". The additional motion can be compensated by translating the patient support surface along the X and/or Z axes in the opposite direction to the additional motion introduced during the roll movement. In other implementations, a pitch rotation movement according to an alternative mechanism may introduce additional motion in any one or more of the X, Y, and Z coordinates.

In each of the examples of figure 18a and figure 18b, a 5 mm standard positioning error is also shown. The 5 mm value is an exemplary value that represents a "couch shift" observed when a patient positioning apparatus is installed and used in a particular clinical setting. In the example of figure 18a, a couch shift of 5 mm in the Y direction is shown and is taken into account for estimating, and compensating, the positioning error in the Y direction. In the example of figure 18b, a couch shift of 5 mm in the X direction is shown and is taken into account for estimating, and compensating, the positioning error in the X direction. In other examples, a couch shift in the Z direction may be taken into account. A couch shift in a particular direction may be related to a load experiencing a centre of gravity shift, which may occur during a particular form of motion during positioning of the load.

Analogous calculations can be performed to characterise a position discrepancy caused by a yaw rotation mechanism such as those described herein. A total unintended shift in each of the X, Y, and Z directions can be calculated for a given patient positioning system by summing each component in each the respective direction, including the couch shift.

It will be appreciated that the geometry of figure 18a and/or figure 18b can be scaled according to any patient positioning system dimensions and any maximum rotation ranges in order to estimate the position discrepancy that may need to be compensated in order to improve the accuracy of patient positioning. Similarly, a method of estimating the accuracy may comprise identifying or characterising a position discrepancy for any given pitch, yaw, or roll angle, and not merely for the maximum of the rotation mechanism range. The patient positioning system may then be controlled with an improved accuracy by compensating any unintended additional motion at any given rotational position. The compensation translation movement may be performed after completion of the rotational pitch, and/or roll, and/or yaw movement, or during that movement. Similarly, the necessary compensation movement may be deduced by a calibration process and stored as a preset value to be implemented. By quantifying the position discrepancy caused by each rotation mechanism and compensating for it by using linear translation motion, the magnitude of the positioning error in each dimension caused by the pitch and roll rotation is made dependent only upon the measurement devices used to measure the position and the measurement devices used to measure the pitch and roll angle. Hence, the overall accuracy of the system is improved.

In particular, a measurement device such as an encoder or an absolute linear scale may be used to measure X, Y, and Z axis translation motion and the resultant position of the patient support surface, and hence patient. Such an encoder may have a measurement accuracy of ± 0.005 mm and an absolute linear scale may have a measurement accuracy of ±0.04 mm. By compensating X, Y, and Z position errors caused by rotational pitch and roll mechanisms, those errors become dependent upon the accuracy to which the pitch or roll angle can be determined, which is improved compared to the non-compensated position error. Such a determination may be made using a suitable sensor, such as the sensors of figures 9-12, or by using an inclinometer. An inclinometer with a measurement accuracy of ± 0.01 degrees may be used. A patient positioning system characterised and/or controlled using the methods disclosed herein can therefore produce highly accurate positioning.

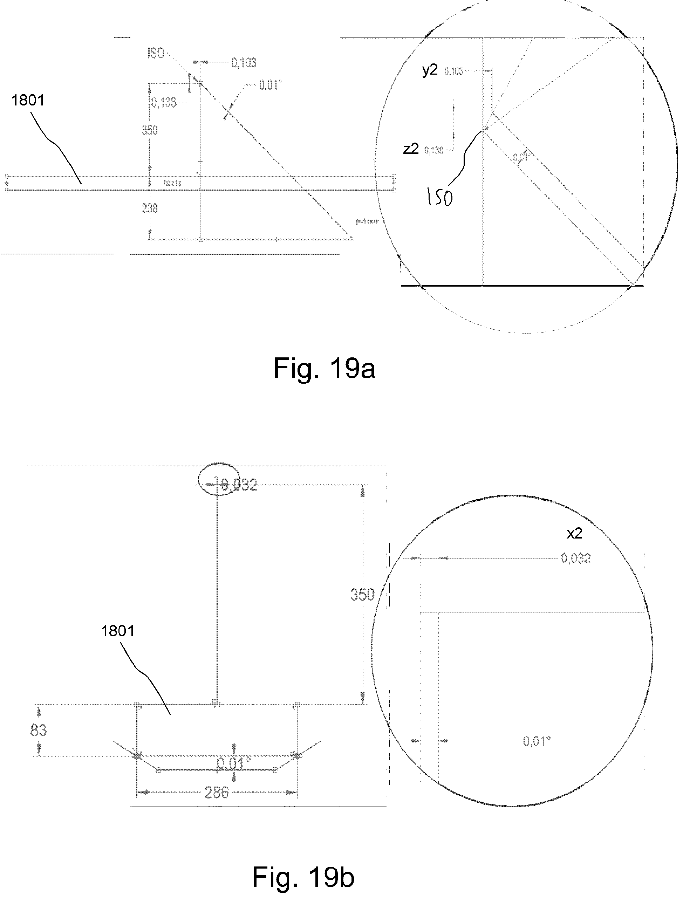

Figure 19a shows a schematic depicting the remaining positioning error, or uncertainty, related to pitch rotation, after the discrepancy shown in the arrangement of figure 18a has been compensated. By using compensatory linear translation motion as described above, the positioning error in the Y and Z directions has been reduced to being dependent upon the accuracy to which the pitch angle can be measured (± 0.01°), as can be seen in the enlarged close-up shown in a circle. The remaining error components, labelled y2 and z2, are 0.103 mm and 0.138 mm respectively for these exemplary dimensions of the patient positioning system.

Figure 19b shows a similar improvement to the accuracy of the roll rotation geometry of figure 18b. Using the compensation approach described above, the error in X position due to roll motion, x2, has been reduced to 0.032 mm. The error in the Z position due to roll motion, z3, has been eliminated entirely to 0 mm.

Table 1 outlines three sources of error, or uncertainty, that may be characterised for the patient support apparatus(es) disclosed herein when using an exemplary geometry and when compensating for unintended motion introduced by the pitch and roll rotation mechanisms. Each component of error can be labelled, such as a Y axis error due to a linear translation being labelled as y1, and given an exemplary uncertainty value corresponding to its exemplary measurement device. A further Y axis error component is labelled y2 and is the remaining error after the pitch rotation Y axis error has been compensated as described above. The three sources of error considered in table 1 relate to linear translation motion error, pitch rotation error, and roll rotation error. The errors y2, z2, and x2, are shown on figures 19a and 19b. The errors x1, y1, and z1 correspond to the accuracy of position measurement related to the linear translation mechanisms of the patient positioning system in each respective direction.

**Table 1: accuracy parameters for various motion components after correction by calibration or pre-set compensation.**

| **Source of error** | **X axis error components (label, and quantity in mm)** | **Y axis error components (label, and quantity in mm)** | **Z axis error components (label, and quantity in mm)** | **Measurement device(s)** |
|---|---|---|---|---|
| X, Y, Z axis linear translation motion (mm) | x1: 0.04 | y1: 0.04 | z1: 0.04 | X and Y position measurement uses an absolute linear scale sensor: ±0.04 mm. |
| | | | | Z position measurement uses an encoder: ±0.005 mm. |
| Pitch Rotation | *Not present.* | y2: 0.103 | z2: 0.138 | Inclinometer: ±0.01°, converted to Y and Z motion accuracy as per figure 19a. i.e., once compensated, the accuracy is set by the inclinometer. |
| Roll Rotation | x2: 0.032 | *Not present.* | z3: 0 | Inclinometer: ±0.01°. |
| | | | | Converted to X and Z motion accuracy as per figure 19b. |

The y2 and z2 error components may be characterised by converting the accuracy of the inclinometer into a y-axis and z-axis accuracy. In this example, the ±0.01° accuracy of measurement results in a y2 value of 0.103 mm and a z2 value of 0.138 mm. It will be appreciated that this method of characterisation can be applied more generally to inclinometers, or other sensors, including those of figures 11a-12, with different measurement accuracy. The accuracy of the x2 and z3 components once compensated for is dependent upon the inclinometer accuracy. Indeed, the z3 component can be reduced to zero by applying appropriate compensation motion.

The overall isocentric positioning accuracy of the system is given by the equation: $Isocentric accuracy = \sqrt{{\left(ΣX\right)}^{2} + {\left(ΣY\right)}^{2} + {\left(ΣZ\right)}^{2}}$

Where Σ*X* is the sum of each source of error, or deviation, in the X direction, e.g. x1 + x2 + x3, ΣY is the sum of each source of error, or deviation, in the Y direction, and Σ*Z* is the sum of each source of error, or deviation, in the Z direction.

The isocentric accuracy can also be considered as a sphere described in the X, Y, and Z directions and it is generally desired that the isocentric accuracy be limited to within as small a sphere as possible. For example, some applications require that isocentric accuracy shall be within a 0.5 mm radius sphere. According to equation (1), the accuracy will be negatively affected by unintended errors, offsets, deviations, discrepancies, or shifts in each of the X, Y, and Z directions, each of which should be reduced as far as possible.

Further sources of error in each direction, which may also be used in equation 1, will now be discussed. There are generally two types of deviation in each of the X, Y, and Z directions: positioning errors related to mechanism accuracy, such as those discussed above, and errors related to rigid deformation of the patient support apparatus and/or patient support surface. In addition, other types of deformation, such as non-rigid deformation, may produce positioning errors.

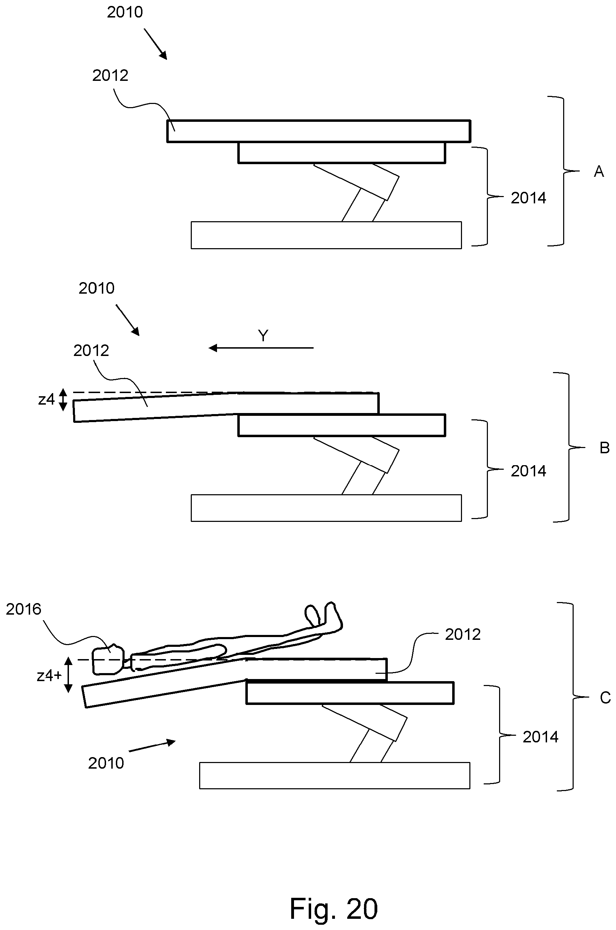

Figure 20 shows example position discrepancies that can arise due to rigid deformation of the patient positioning apparatus in the presence of a patient. A patient positioning system 2010 is shown, which may be equivalent or similar to any of the patient positioning and/or support systems disclosed herein, such as those of figures 1-17b. The patient positioning system 2010 comprises a patient support surface 2012 and positioning apparatus 2014, such as those described herein. The patient positioning system 2010 is shown in a first arrangement A in which the patient support surface 2012 is in a first position. In a second arrangement, B, the patient support surface 2012 has been moved by the positioning apparatus 2014 in the Y direction indicated by the single headed arrow labelled Y. The patient support surface 2012 has been moved by linear translation in the Y direction, but due to the weight of the patient support surface 2012 being less supported by the positioning apparatus 2104 in that position than in the position of the first arrangement A, the patient support surface 2012 experiences rigid deformation, or deflection, or bending, downwards. The degree of deformation is greatly exaggerated in the figure for the purpose of illustrating the effect. The rigid deformation produces a discrepancy between the expected Z position of the patient support surface 2012 and the actual Z position of the patient support surface 2012. A dashed line shows the intended Z position of the patient support surface 2021 and the discrepancy is indicated by the double-headed arrow labelled z4. The position discrepancy due to rigid deformation from a change in Y position of the patient support surface is thus referred to as z4. In some examples, positioning discrepancy due to rigid deformation may increase in proportion to the distance in the Y direction and/or X direction that the patient support surface is moved away from the support base of the positioning apparatus 2014.

In a third arrangement shown in figure 20 and labelled C, a patient 2016 is present on the patient support surface 2012, which, like the second arrangement B, is extended in the Y direction. The weight of the patient 2016 can cause rigid deformation of the patient support surface 2012 or the weight of the patient 2016 can exacerbate existing rigid deformation of the patient support surface 2012, such as that of the second arrangement B, increasing the position discrepancy z4. The rigid deformation of the patient support surface 2012 in the third arrangement C is thus indicated with a dashed line and a double-headed arrow labelled z4+. Rigid deformation may also be referred to as sag, or distortion, or deflection, or bending. The rigid deformation shown in figure 20 is shown using an exaggerated schematic to aid understanding, and may not be so significant a proportion of the height of the patient positioning system in actual effect.

Overall, four forms of rigid deformation can be identified for the patient positioning systems disclosed herein, and are listed in table 2. In addition to deformation due to Y motion that causes a discrepancy in Z position (z4), as described in the example of figure 20, analogous Z position deformation can occur due to X motion (z5 in table 2). Furthermore, rigid deformation can cause discrepancies in the X and Y position of the patient support surface, and hence the patient position. The X and Y position can vary as a result of rigid deformation exhibited during rotation of the patient support surface, and each discrepancy is labelled x3 and y3 respectively. Such variation may be referred to as isocentre rotation breakdown and may occur due to the table or patient weight being shifted away from the intended centre of gravity. That in turn may cause a shift or tilt of the patient positioning system away from the intended yaw rotation axis, causing a shift in the isocentre in the X and/or Y directions, or in polar coordinates if viewing the yaw rotation axis of the patient positioning system from above. Likewise, the X and Y position can vary as a result of deformation caused by movement of the patient support surface in the Z direction, with each discrepancy respectively labelled as x4 and y4. Each of these discrepancies must be corrected or compensated to produce a reduced error.

Exemplary values of the reduced error due to each type of deformation are given in table 2. It will be understood that systems with varying geometry or different measurement devices or sensors will have varying error. In some systems, the system may be designed such that within an expected range of motion, no deformation error is present. However, if the system travels outside its normal range of motion to compensate for positioning errors such as those described above, the components of the system may undergo rigid deformation or sag. For example, the "Z motion deformation" of table 2 may be a deformation in X and Y position that is caused due to the Z positioning mechanisms having to produce an additional 47 mm of travel in order to compensate for the error described in the example of figure 18a (42 mm of error due to the rotational mechanism added to 5 mm of error due to couch shift, producing a possible shift of 47 mm in Z position). Failure to account for such deformation can result in low accuracy or high error in the system even when compensating for particular types of positioning error.

Each of the position discrepancies caused by each type rigid deformation can be caused and/or exacerbated by the presence of a patient on the patient support surface.

**Table 2: accuracy parameters for various motion components after correction by calibration or compensation**

| **Source of error** | **X axis error components (label, and quantity in mm)** | **Y axis error components (label, and quantity in mm)** | **Z axis error components (label, and quantity in mm)** | **Further comment** |
|---|---|---|---|---|
| Isocentre rotation deformation | x3: 0.04 | y3: 0.04 | *Not present.* | Error remaining after compensation by calibration or a pre-set compensation factor. |
| Z motion deformation | x4: 0.028. | y4: 0.031 | *Not present.* | Error remaining after compensation of deformation in X, Y due to extra 47 mm Z travel for compensation. |
| Y motion deformation | *Not present.* | *Not present.* | z4: 0.18 | Error remaining after compensation of deformation in Z due to extra 40 mm Y travel for compensation. |
| X motion deformation | *Not present.* | *Not present.* | z5: 0.06 | Error remaining after compensation of deformation in Z due to extra 15 mm X travel for compensation. |

The accuracy of the system according to equation (1) hence depends on the values for each discrepancy x1, x2, x3, x4, y1, y2, y3, y4, z1, z2, z3, z4, z5, contained in Table 1 and Table 2.

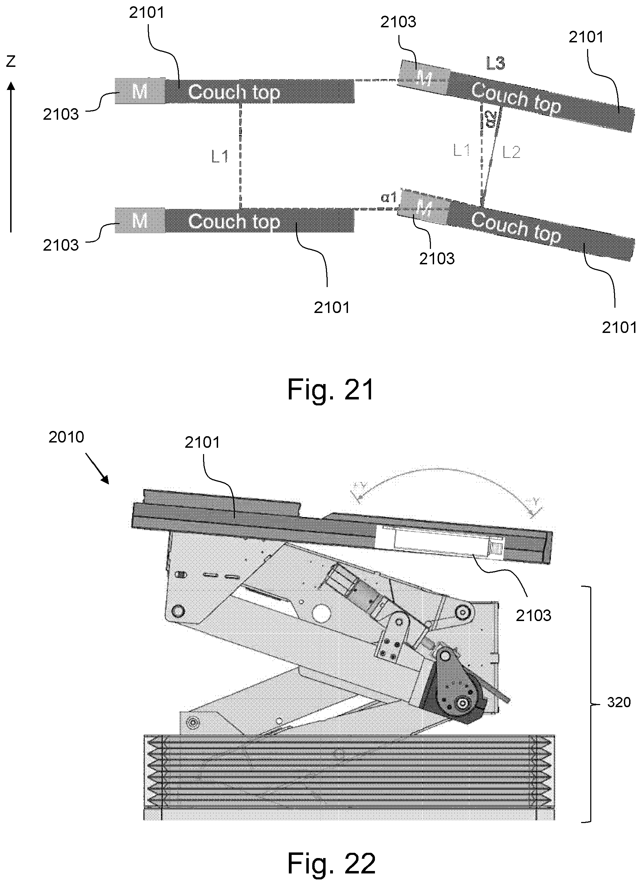

Figure 21 shows a schematic of a patient support surface 2101 that exhibits deformation, or sag. The patient support surface may be equivalent or similar to any of the patient support surfaces described herein. At least one inclinometer 2103 is mounted to, attached, or embedded in the patient support surface 2101. The patient support surface 2101 is shown in four positions. In the position shown in the bottom left of figure 21, the patient support surface 2101 has no tilt, or incline, and is in a low position along the Z axis. In the top left of the figure, the patient support surface 2101 has translated such that it is raised to a higher position along the Z axis, as indicated by the single headed arrow labelled "Z". The patient support surface 2101 is thus at a height L1 and each point on the patient support surface 2101 will be raised by and to the height L1 without also experiencing any unintended motion in the X, Y, or Z directions.

In the position shown in the bottom right of figure 21, the patient support surface 2101 has been rotated such that it is tilted with respect to its horizontal position by an angle α1. If the patient support surface 2101 is raised while in that tilted position, and translated through a height L1 like on the left hand side of figure 21, a point on the surface of the patient support surface 2101 may unintentionally shift in the X and/or Y direction due to bending. The equivalent translation of the point on the surface is represented by the length L2, which is not parallel to L1 and is offset from L1 by an angle α2. In this example, the shift produced by L2 being offset from L1 by the angle α2 is in the Y direction and is represented by the length L3. L3 is determined during manufacture of the patient positioning system, and typically has a maximum value of 3.7 mm for Z axis travel of 1000 mm with a heavy load. However, the magnitude of L3 for Z axis travel over a smaller distance may be much smaller, and for Z axis travel of 47 mm, L3 is typically around 0.174 mm for systems disclosed herein. The angle α2 is determined during manufacture of the patient positioning system, and typically has a small value. For example, for a Z travel of 1000 mm and L3 value of 3.7 mm, α2 is around 0.25 degrees for systems disclosed herein. α2 is a property of structure variance.

The inclinometer 2103 can be used to determine the bending deformation angle in the X and/or Y directions. In some examples, an alternative sensor such as a clinometer or tilt sensor may be used, or the sensors of figures 11-12. As an example, considering an overall compensation shift in the Z direction of 47 mm, an inclinometer reading (*I*) in the X and/or Y directions can be obtained after 47 mm of travel and used to compensate the bending error L3. The inclinometer reading (*I*) represents a measured tilt experienced by the patient support surface after it is translated through the distance of 47 mm in the Z direction. As with the approach to compensating positional discrepancies described above, the position error due to the bending error L3 can be compensated, leaving a remaining error L3'. The remaining error L3' (in millimetres) is given by the equation: $L 3 ′ = L 3 - 47 sin \left(I\right)$

In other examples, the 47 of equation (2) is substituted with an appropriate alternative value representing the distance of motion in the Z direction.

L3' itself can be converted to give an improved error, or accuracy, for the X and Y direction components x4 and y4 according to the following equations: $\frac{y 4}{L 3 ′} = \frac{47}{L 1}$ $\frac{x 4}{L 3 ′} = \frac{47}{L 1}$

Exemplary values for y4 and x4 after compensation were found to be 0.031 mm and 0.028 mm respectively, as given in table 2.

An inclinometer can also be used to detect the discrepancy due to bending in the Z axis that occurs during motion of the patient support surface in the Y direction and/or the X direction. If a patient is present on the patient support surface, the weight of the patient will typically exacerbate bending in the Z direction as the patient support surface position moves further from the support structure 320 in a Y direction, or an X direction.

Figure 22 shows a patient positioning system 2010 with an inclinometer 2103 mounted to it. The inclinometer 2103 is arranged to measure the tilt or inclination in the Y axis, also known as the pitch of the table or patient support surface, which is indicated by the curved line labelled at either end as +Y or -Y. Similar inclinometers can be used to measure rotation in the X axis, known as roll rotation, or even to measure the Z axis yaw rotation. In some implementations, a multi-axis inclinometer is used to measure the angle of rotation or tilt along one or more directions.

In some implementations, one or more inclinometers are used to measure the position of the patient support surface. In some examples, a dual axis inclinometer is placed at an appropriate location in order to measure the pitch and roll angles of the patient support surface. On one of its axes, the inclinometer measures an absolute value for the pitch angle of the patient support surface, and on the other of its axes, the inclinometer measures an absolute value for the roll angle. In some examples, an additional inclinometer can be used to measure the yaw, or heading, rotation of the patient support surface as an absolute angle value. Alternatively, a single electronic compass, or a three axis inclinometer, may be used to provide a measure of the absolute angle of pitch, roll, and yaw of the patient support surface. Whether one or more inclinometers or electronic compasses are used, multiple alternative or complementary mounting locations are possible. In some examples, such as that of figure 22, an inclinometer or electronic compass is mounted to the patient support surface.

In some examples, an inclinometer or electronic compass is mounted directly under the patient support surface. In examples in which multiple inclinometers are used, one inclinometer, or electronic compass, may be mounted to the side of the patient support surface and another mounted directly under the treatment table. Any mounting location which allows the determination of the desired angle may be used. Whichever configuration is used, the inclinometer 2103 is located and oriented appropriately to measure the angle desired to be measured, for example, by orienting an axis of the inclinometer to measure the roll angle of the table. The positioning of the inclinometer thus enables the measurement of tilt and/or roll that enable the approaches to improve accuracy of patient positioning disclosed herein.

Known techniques measure the movements of patient positioning systems using linear or rotation encoders, which provide a value of the mechanical movement required for a particular roll and pitch. However, such measurements are influenced by factors such as mechanical tolerances and do not measure roll and pitch directly. Advantageously, an inclinometer measures the absolute value of the roll, pitch, and/or yaw, providing the actual position of the table even in instances of bending or deformation, such as that due to the presence of a patient.

Furthermore, by using inclinometers mounted to the patient support surface, the movement of the table can be measured at the table itself, which is more accurate than a solution where the pitch is measured at the pitch articulation and control mechanism. Furthermore, obtaining an absolute measurement means that the horizontal calibration of the table can be set at installation by using the inclinometer measurements.

In implementations wherein a patient is positioned on the patient support surface and the weight of the patient causes deformation, bending, and/or sag in the Z direction, the deformation may be proportional to the weight of the patient. In such implementations, the inclinometer 2103 will measure the bending deformation of the patient support surface.

The inventors have appreciated that there is a linear relationship between the weight of the patient and a measurement of bending obtainable at the inclinometer 2103. In particular, a parameter k can be assigned to the inclinometer measurement and the parameter k has a linear relationship with patient weight.

The approaches described herein can be used to calculate and compensate for bending of the patient support surface in the Z direction caused during additional motion of the patient support surface in the Y direction, the additional motion in the Y direction being performed in order to compensate for an offset in the position of the patient support surface in the Y direction.

For example, if an additional 40 mm of travel is needed to compensate for positional errors in the Y direction introduced during rotation, an inclinometer measurement can be taken over 40 mm of travel in order to detect a change in inclination ΔI. ΔI can be used to estimate the corresponding bending deformation of the patient support surface. For Y motion, the inventors have appreciated that the parameter k is set by the ratio of the inclinometer reading in the Y direction when the patient is present (Δ*I_{patient load}*) to the inclinometer reading in the Y direction under a load of 30 kg (Δ*I*_{30 *kg*}): $k = Δ {I}_{patient load} / Δ {I}_{30 kg}$

In defining the parameter k, the change in inclinometer reading for the patient load and for a load of 30 kg should be taken over the same distance. Typically the distance should be larger than 100 mm. For example, Δ*I_{patient load}* and Δ*I*_{30 *kg*} may each be measured during travel from 0 to 500 mm. In one example, the distance is the range of motion in the Y direction. In some examples, the parameter k can be calibrated such that it can be estimated using the expected deflection due to the weight of the patient. The weight of the patient, or patient load, may be measured or estimated and a corresponding Δ*I_{patient load}* may be measured or estimated. Δ*I_{patient load}* is thus a parameter based on, or derived from, or dependent on a physical characteristic of the patient, such as weight.

Another parameter, b, can be determined according to tests conducted during manufacturing. The parameter b is determined from measuring a series of data points for sag discrepancy at the pitch rotation centre shaft when the system is under 30kg load. The series of data points is taken such that each data point represents an incremental 20 mm step of travel in the Y direction. The parameter b is measured (for example, using a dial indicator) as a benchmark during manufacturing production of each patient positioning system and may be stored at the system, such as in computer memory as a look-up table that stores the parameter b as a function of Y travel. In some examples, the parameter b is small enough that it can be considered to be equal to zero. The position discrepancy in the Z direction due to Y travel, *Z_{cal,y},* can be calculated with: ${Z}_{cal , y} = R sin \left(ΔI\right) \pm kb$

Where R is the distance of the radiation delivery isocentre from the pivot around which the tilt motion is produced, described above as the centre of pitch rotation. Using *Z_{cal,y}* from equation 6 to offset bending error using translational motion in the Z direction, the maximum remaining error in Z position z4 caused by bending deformation from motion in the Y direction is found to be 0.181 mm. This improved accuracy is achieved by using a measurement or estimate of the patient weight in order to estimate the parameters Δ*I_{patient load}* and *k,* or by measuring Δ*I_{patient load}* directly. For example, a calibration of the parameter k at different patient loads may be performed and stored in a computer memory as a look-up table. The measured or estimated patient weight may then be used with the look-up table in order to estimate the parameter *k.* Alternatively, the parameter *k* may be determined from inclinometer readings during the patient positioning process, such as by measuring Δ*I_{patient load}* when a patient is moved using the patient support surface through a distance of, for example, 100 mm. Such an approach allows the patient positioning system to efficiently improve positioning accuracy based on the individual patient and position.

The position discrepancy z4 that arises due to bending or deformation may be compensated using linear translational motion, as described above, or may be compensated at least partly using rotational motion. Using rotational motion rather than linear translational motion to compensate a position discrepancy may in some examples improve the overall positioning accuracy of the systems disclosed herein. For example, a typical position accuracy for linear motion position in the Z direction is 0.04 mm, as shown in table 1. Using the system geometries and methods disclosed herein to compensate sag using linear motion in the Z direction leaves a remaining error (z4) of 0.181 mm, as shown in tables 2 and 3.

A typical measurement accuracy for tilt angle, and hence Z position at a particular tilt angle, is 0.01°. However, an inclinometer sensor that has 0.01° absolute accuracy may in fact have a repeatability of +/-0.003°, according to tests performed by the inventors. The highly precise repeatability of the tilt angle can thus be used in order to improve the overall accuracy of the Z coordinate positioning.

In some examples, when the patient is loaded onto the patient support surface, an initial inclinometer reading is taken in the Y axis direction. When the patient support surface is moved in the Y direction, for example over the course of a Y direction compensation motion (over an exemplary distance of 37 mm), bending or sag of the upper structure will result in a position discrepancy and a change to the inclinometer reading. The patient positioning system is then controlled by using pitch motion to rotate the patient support surface to the position at which the inclinometer reading matches the initial reading, which is the intended position. Pitch rotation is thus used to compensate an offset in the Z direction by making use of the inclinometer repeatability of +/-0.003°.

In some examples, sag during or due to travel in the Y direction may also affect the pitch rotation axis, so an additional element of compensatory Z axis motion is required in order to accurately compensate for Z positioning error by using pitch rotation. In such examples, the required amount of compensatory Z axis motion is given by the equation *Z_{cal,pitch} = k*Δ*Z*_{(30*kg*)}, where *k* is according to equation (5) and Z is the absolute height of the patient support surface above the ground, which will be affected by deformation of the patient support surface. In order to compensate this change in Z, the change in Z when the patient support surface is under 30kg load (Δ*Z*_{(30*kg*)}) is measured for incremental steps in Y travel during manufacturing production of the patient positioning system. The data may be stored with each individual patient positioning system, such as by storing a lookup table in a computer memory. When the patient positioning system is used in a clinical scenario, a patient is loaded onto the patient positioning system and the k value for the patient is calculated. For each motion in the Y direction, or Y position, Δ*Z*_{(30*kg*)} can be retrieved from the look up table and *Z_{cal,pitch}* can be calculated. The remaining error z4 once also compensated by *Z_{cal,pitch}* is as little as 0.081 mm, an improvement over the 0.18 mm value for z4 that is achieved by compensation using linear translation motion. With a value of 0.081 mm for z4, an overall system positioning accuracy (according to equation 1) within a 0.403 mm radius sphere can be achieved, well within the 0.5 mm requirement.

Furthermore, in each example, the weight of the patient may in fact be measured by using the inclinometer or other sensors that are used to measure the inclination and/or position of the patient support surface. For example, the parameter Δ*I_{patient load}* may simply be measured by the inclinometer with the patient present on the patient positioning system, rather than being determined by calibration or estimated in the absence of the patient. In each case, tailoring the control of the patient positioning system to individual patient physical characteristics allows the patient positioning system to move the patient to the correct position more accurately and more quickly.

An analogous calculation of compensation value can be performed for the error in Z position z5 that remains after compensating bending deformation from motion in the X direction. In such examples, the at least one inclinometer is arranged such that it can measure the bending deformation for movement along the X direction. An additional measure of position in the X direction, (*Xₐ - A*), is introduced into the calculation of *Z_{cal}* in the X direction, as follows: ${Z}_{cal , x} = \left({X}_{a}-A\right) tan \left(ΔI\right) \pm kb$

Where *A* is the maximum travel range of the patient support surface in the X direction and *Xₐ* is the position of the patient support surface relative to a fixed point at which it is best, or well, supported. In an example, *A =* 125 *mm.* In an example, the fixed point at which the patient support surface is best supported is at the centre of its range of motion. In some examples, the sag in the Z direction according to X position, *Z_{cal,x},* is asymmetric about the fixed point at which the patient support surface is best supported. If the patient support surface is positioned directly over the support apparatus, *Xₐ* will be equal to A and no bending deformation will be expected. As the patient support surface moves away from that position, the difference between *Xₐ* and *A* will increase and bending deformation will occur according to *Z_{cai,x}.* The inventors have appreciated that in some examples of patient positioning systems, such as those disclosed herein, k = 0 for bending deformation due to motion in the X direction, corresponding to a situation in which the patient's weight does not affect the amount of Z position bending deformation caused by motion in the X direction. However, in other examples, k will take a non-zero value, and the patient's weight will affect the amount of Z position bending deformation caused by motion in the X direction. In equation (7), Δ*I* is the detected change in the inclinometer reading as the patient support surface moves in the X direction.

Using *Z_{cal,x}* from equation 7 to offset bending error, the maximum remaining error in Z position caused by bending deformation from motion in the X direction is found to be 0.0525 mm.

Each of the separate sources of error and position discrepancy described herein is summarised in table 3. Using the systems and approaches disclosed herein, the overall isocentric accuracy of the patient positioning system, as given by equation (1) above, is defined by a sphere with a radius of 0.491 mm, meeting the requirement of a radius of less than 0.5 mm.

**Table 3: summary of exemplary improved accuracy enabled by the systems and methods described herein**

| **Source of error** | **X axis accuracy (mm)** | **Y axis accuracy (mm)** | **Z axis accuracy (mm)** | **Sensor or component that accuracy is determined by** |
|---|---|---|---|---|
| X, Y, Z axis linear translation motion | x1: 0.04 | y1: 0.04 | z1: 0.04 | X, Y Linear scale: ±0.04mm |
| | | | | Z encoder: ±0.005mm |
| Pitch rotation | *Not affected.* | y2: 0.103 | z2: 0.138 | Pitch inclinometer: ±0.01° converted to X, Y motion accuracy. |
| Roll rotation | *x2: 0.032* | *Not affected.* | z3: 0 | Roll inclinometer: ±0.01° converted to X, Z motion accuracy. |
| Deformation from isocentre rotation | x3: 0.04 | y3: 0.04 | *Not affected.* | 0.354mm offset by calibration or preset compensation. |
| Z motion deformation | x4: 0.028 | y4: 0.031 | *Not affected.* | After compensating deformation in X, Y due to extra 47mm Z travel for compensation. |
| Y motion deformation | *Not affected.* | *Not affected.* | z4: 0.181 (or 0.081 if using pitch rotation to compensate) | After compensating deformation in Z due to extra Y travel for compensation. |
| X motion deformation | *Not affected.* | *Not affected.* | z5: 0.06 | After compensating deformation in Z due to extra X travel for compensation. |
| ***Maximum X, Y, Z error in total*** | *ΣX: 0.14* | *ΣY: 0.214* | *ΣZ: 0.419* | - |
| ***Square sum root (overall isocentric accuracy according to equation 1)*** | *0.491 (or 0.403 if using pitch rotation to compensate z4)* | | | - |

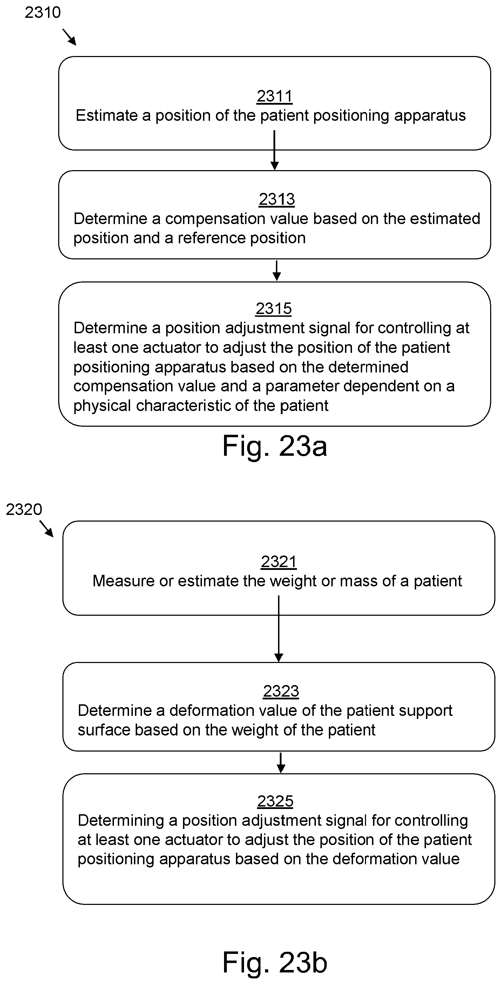

Figure 23a shows a method 2310 according to the present disclosure. At a first block 2311, the position of a patient positioning apparatus (such as the patient support surfaces disclosed herein) is estimated. The position may be estimated based on a signal from at least one sensor, such as the inclinometer 2103 of figure 22. At a second block 2313, the estimated position is compared with the intended position, or coordinates, of the patient positioning apparatus, which is referred to as a reference position. The reference position may be determined by a radiotherapy treatment plan, which in turn is determined based on the patient's clinical needs. For example, the reference position may be a position of the patient positioning apparatus which will bring a region of the patient's internal anatomy (such as a tumour) into an isocentre of the radiotherapy device. The reference position may be a position of the patient positioning device at which a particular region of the patient's anatomy (such as an organ at risk) will avoid radiation via the beam of therapeutic radiation. The radiotherapy treatment plan specifies a number of other treatment parameters which affect treatment, such as a number of beams (beamlets) to be applied per fraction, angles from which the beams will be applied, the dose per beam, the beam shapes, etc. The reference position may be fixed throughout treatment, or may be varied for different beams of the treatment.

A compensation value, which may be equivalent to a discrepancy between the estimated position and the reference position, is determined. The compensation value can be a vector which describes a difference between the estimated position and the reference position. Depending on the implementation, the compensation value may be a three dimensional vector which describes the X, Y and Z adjustments required to bring the estimated position into alignment with the reference position.

At a third block 2315, the method comprises determining a position adjustment signal for controlling at least one actuator to adjust the position of the patient positioning apparatus based on the determined compensation value and a parameter derived from, or dependent on, or based on, a physical characteristic of the patient. The at least one actuator is configured to implement the position adjustment signal in order to bring the position of the patient positioning apparatus into alignment with the reference position, such that the patient is positioned optimally for radiotherapy and according to their treatment plan. The position adjustment signal is determined using not only the compensation value, but also the patient parameter. To generate the position adjustment signal, the compensation value is modified by the patient parameter such that the deformation caused by the patient's weight is taken into account during the corrective movement. Accordingly, the patient positioning apparatus can be brought into close alignment with the reference position, with high accuracy.

If the position adjustment signal were instead calculated based solely on the difference between the estimated position and the reference position, i.e. if effects associated with deformation of the apparatus due to the patient's weight were not taken into account, this may result in a continued discrepancy between the actual position of the patient positioning apparatus and the reference position. The present methods therefore provide a more accurate method and system for controlling a patient positioning apparatus. Adjustments calculated according to the present methods are more likely to be accurate first time, and do not require time-consuming continued adjustments according to feedback from e.g. a camera or other position monitoring system. Accordingly, the present methods is not only more accurate, but also more timely.

Figure 23b shows a method 2320 according to the present disclosure. The method 2320 may comprise further steps and/or features not shown in the figure, for example additional steps disclosed in connection with any of the approaches described herein. At a first block 2321, the weight or mass of a patient is estimated or measured, as described above. Any of the examples described above can be used, such as estimating the weight of the patient, obtaining the patient's weight from their medical records, or such as using an inclinometer on the patient positioning system to measure or infer the weight of the patient. For example, the weight of the patient may be implicit in the parameter Δ*I_{patient load}* which may be determined from signals produced by the inclinometer. At a second block 2323, a deformation value of the patient support surface is determined based on the measured, estimated, or inferred weight of the patient, for example by using at least one of equations 5 to 7. At a third block 2325, the method comprises determining a position adjustment signal for controlling at least one actuator to adjust the position of the patient positioning apparatus based on the determined deformation value.

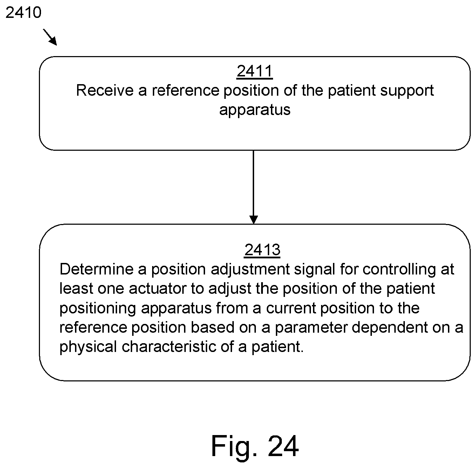

Figure 24 shows a method 2410 according to the present disclosure. The method 2410 may comprise further steps and/or features not shown in the figure, for example additional steps disclosed in connection with any of the approaches described herein. At a first block 2411, a reference position of the patient support apparatus is received. The reference position is the intended position to which the patient support apparatus is to be moved. At a second block 2413, a position adjustment signal is determined. The position adjustment signal is for controlling at least one actuator to adjust the position of the patient positioning apparatus from a current position to the reference position. The position adjustment signal is determined based on a parameter dependent on a physical characteristic of a patient. Any of the examples described above can be used, such as estimating the weight of the patient, obtaining the patient's weight from their medical records, or such as using an inclinometer on the patient positioning system to measure or infer the weight of the patient. For example, the weight of the patient may be implicit in the parameter Δ*I_{patient load}* which may be determined from signals produced by the inclinometer.

Each method may be performed individually, or the methods may be performed in combination. The overall control of the patient positioning system may make use of all three of, or a combination of two of, the methods of Figs. 23a, 23b, and 24, and each may apply to one or more of the X, Y, and/or Z directions. In other examples, position discrepancy due to rotation mechanisms may not be present and only discrepancy and compensation based on deformation may be considered. Each method, or a combination of methods, may be implemented using any of the systems or apparatus disclosed herein, such as the patient support apparatus and/or patient positioning apparatus of Figs. 1 to 22.

In some examples, an alternative physical characteristic of a patient to patient weight may be used, such as patient height, or a combination of weight and height may be used.

Optionally, the method(s) comprise determining a position adjustment signal such that an overall isocentric positioning accuracy of the patient positioning device corresponds to a sphere with a radius of less than 0.5 mm. The method(s) further optionally comprise characterising the overall isocentric accuracy of the patient positioning system.

Each method, or the combined method, optionally further comprises controlling the at least one actuator using the position adjustment signal.

Further optionally, the method(s) further comprise adjusting a radiotherapy treatment plan in accordance with a characterised accuracy of the patient positioning system, and/or optimising a radiotherapy treatment plan in accordance with a characterised accuracy of the patient positioning system.

In some examples, the position adjustment signal is arranged to compensate for the weight of a patient.

The term "computer-readable medium" as used herein refers to any medium that stores data and/or instructions for causing a processor to operate in a specific manner. Such storage medium may comprise non-volatile media and/or volatile media. Non-volatile media may include, for example, optical or magnetic disks. Volatile media may include dynamic memory. Exemplary forms of storage medium include, a floppy disk, a flexible disk, a hard disk, a solid state drive, a magnetic tape, or any other magnetic data storage medium, a CD-ROM, any other optical data storage medium, any physical medium with one or more patterns of holes, a RAM, a PROM, an EPROM, a FLASH-EPROM, NVRAM, and any other memory chip or cartridge.

It will be understood that the above description of specific embodiments is by way of example only and is not intended to limit the scope of the present disclosure. Many modifications of the described embodiments are envisaged and intended to be within the scope of the present disclosure.

Arrangements of the present disclosure described above are only exemplary and many variations are possible, as will be apparent to the skilled person. Thus, the above description comprises examples comprising preferred and/or optional features of the disclosed arrangements, strict literal compliance with the meaning of the words is not intended and there may be other variations apparent to the skilled person that result in substantially the same or similar effects.

## Claims

1. A computer-implemented method (2410) of controlling a patient positioning apparatus for a radiotherapy system, the patient positioning apparatus comprising a tiltable patient support apparatus, the method (2410) comprising:
receiving (2411) a reference position of the patient support apparatus;
determining (2413) a position adjustment signal for controlling at least one actuator to adjust the position of the patient positioning apparatus from a current position to the reference position based on a parameter dependent on a physical characteristic of a patient,
wherein the position adjustment signal is further based on a discrepancy between an estimated position and the reference position,
wherein the estimated position is estimated at least partially based on a reading from a sensor,
wherein the parameter dependent on a physical characteristic of the patient is determined using signals received from the sensor.

2. The method (2410) according to claim 1, wherein the physical characteristic of the patient comprises at least one of patient weight and patient height.

3. The method (2410) according to claim 1 or claim 2, wherein the discrepancy is caused by rotational motion of the tiltable patient support apparatus,
optionally wherein the position adjustment signal is arranged to compensate the discrepancy by causing translational motion of the tiltable patient support apparatus.

4. The method (2410) according to any of claims 1 to 3, wherein the sensor is an inclinometer mounted to the tiltable patient support apparatus.

5. The method (2410) according to any preceding claim, wherein the parameter dependent on a physical characteristic of the patient is determined from the difference between a first sensor reading and a second sensor reading, the first sensor reading taken at a first position of the tiltable patient support apparatus, the second sensor reading taken at a second position of the tiltable patient support apparatus, wherein the patient is positioned on the tiltable patient support apparatus during each sensor reading.

6. The method (2410) according to any of claims 1 to 5, wherein the discrepancy between the estimated position and the reference position is caused by deformation of the patient support apparatus,
optionally wherein the deformation of the patient support apparatus occurs when a patient is present on the patient support apparatus.

7. The method (2410) according to claim 6, wherein the deformation of the patient support apparatus causes a discrepancy between the estimated position and the reference position in a vertical direction.

8. The method (2410) of any preceding claim, wherein the position adjustment signal is arranged to cause pitch rotation motion of the tiltable patient support apparatus.

9. The method (2410) according to any preceding claim, the method further comprising controlling the at least one actuator using the position adjustment signal.

10. The method (2410) according to any preceding claim, wherein the method comprises determining a position adjustment signal such that an overall isocentric positioning accuracy of the patient positioning device corresponds to a sphere with a radius of less than 0.5 mm.

11. The method (2410) according to any preceding claim, wherein at least one of:
A. the position adjustment signal is determined from the parameter dependent on the physical characteristic of the patient by using a look-up table; and/or
B. the position adjustment signal is arranged to compensate for the weight of a patient.

12. A radiotherapy device comprising a processor configured to perform the method (2410) of any preceding claim.

13. A computer-readable medium containing instructions that, when executed by a processor, cause a radiotherapy system to perform the method (2410) of any of claims 1 to 11.

## Patentansprüche

1. Computerimplementiertes Verfahren (2410) zum Steuern einer Patientenpositionierungsvorrichtung für ein Strahlentherapiesystem, wobei die Patientenpositionierungsvorrichtung eine kippbare Patiententragevorrichtung umfasst, wobei das Verfahren (2410) Folgendes umfasst:
Empfangen (2411) einer Referenzposition der Patiententragevorrichtung;
Bestimmen (2413) eines Positionseinstellungssignals zum Steuern mindestens eines Aktuators zum Einstellen der Position der Patientenpositionierungsvorrichtung von einer aktuellen Position in die Referenzposition basierend auf einem von einer körperlichen Eigenschaft eines Patienten abhängigen Parameter,
wobei das Positionseinstellungssignal ferner auf einer Diskrepanz zwischen einer geschätzten Position und der Referenzposition basiert,
wobei die geschätzte Position zumindest teilweise basierend auf einer Ablesung von einem Sensor geschätzt wird,
wobei der von einer körperlichen Eigenschaft des Patienten abhängige Parameter unter Verwendung von von dem Sensor empfangenen Signalen bestimmt wird.

2. Verfahren (2410) nach Anspruch 1, wobei die körperliche Eigenschaft des Patienten das Patientengewicht und/oder die Patientenkörpergröße umfasst.

3. Verfahren (2410) nach Anspruch 1 oder Anspruch 2, wobei die Diskrepanz durch eine Drehbewegung der kippbaren Patiententragevorrichtung bewirkt wird,
wobei das Positionseinstellungssignal optional dazu ausgelegt ist, die Diskrepanz durch Bewirken einer translatorischen Bewegung der kippbaren Patiententragevorrichtung zu kompensieren.

4. Verfahren (2410) nach einem der Ansprüche 1 bis 3, wobei der Sensor ein an der kippbaren Patiententragevorrichtung montierter Neigungsmesser ist.

5. Verfahren (2410) nach einem der vorhergehenden Ansprüche, wobei der von einer körperlichen Eigenschaft des Patienten abhängige Parameter aus der Differenz zwischen einer ersten Sensorablesung und einer zweiten Sensorablesung bestimmt wird, wobei die erste Sensorablesung an einer ersten Position der kippbaren Patiententragevorrichtung vorgenommen wird, wobei die zweite Sensorablesung an einer zweiten Position der kippbaren Patiententragevorrichtung vorgenommen wird, wobei der Patient während jeder Sensorablesung auf der kippbaren Patiententragevorrichtung positioniert ist.

6. Verfahren (2410) nach einem der Ansprüche 1 bis 5, wobei die Diskrepanz zwischen der geschätzten Position und der Referenzposition durch eine Verformung der Patiententragevorrichtung bewirkt wird,
wobei die Verformung der Patiententragevorrichtung optional auftritt, wenn sich ein Patient auf der Patiententragevorrichtung befindet.

7. Verfahren (2410) nach Anspruch 6, wobei die Verformung der Patiententragevorrichtung eine Diskrepanz zwischen der geschätzten Position und der Referenzposition in einer vertikalen Richtung bewirkt.

8. Verfahren (2410) nach einem der vorhergehenden Ansprüche, wobei das Positionseinstellungssignal dazu ausgelegt ist, eine Nickdrehbewegung der kippbaren Patiententragevorrichtung zu bewirken.

9. Verfahren (2410) nach einem der vorhergehenden Ansprüche, wobei das Verfahren ferner das Steuern des mindestens einen Aktuators unter Verwendung des Positionseinstellungssignals umfasst.

10. Verfahren (2410) nach einem der vorhergehenden Ansprüche, wobei das Verfahren das Bestimmen eines Positionseinstellungssignals umfasst, so dass eine isozentrische Gesamtpositionierungsgenauigkeit der Patientenpositionierungsvorrichtung einer Kugel mit einem Radius von weniger als 0,5 mm entspricht.

11. Verfahren (2410) nach einem der vorhergehenden Ansprüche, wobei mindestens eines von Folgendem gilt:
A. das Positionseinstellungssignal wird aus dem von der körperlichen Eigenschaft des Patienten abhängigen Parameter unter Verwendung einer Nachschlagetabelle bestimmt; und/oder
B. das Positionseinstellungssignal ist dazu ausgelegt, das Gewicht eines Patienten zu kompensieren.

12. Strahlentherapieeinrichtung, die einen Prozessor umfasst, der dazu konfiguriert ist, das Verfahren (2410) nach einem der vorhergehenden Ansprüche durchzuführen.

13. Computerlesbares Medium, das Anweisungen enthält, die bei Ausführung durch einen Prozessor bewirken, dass ein Strahlentherapiesystem das Verfahren (2410) nach einem der Ansprüche 1 bis 11 durchführt.

## Revendications

1. Procédé (2410) mis en œuvre par ordinateur de commande d'un appareil de positionnement de patient pour un système de radiothérapie, l'appareil de positionnement de patient comprenant un appareil de support de patient inclinable, le procédé (2410) comprenant :
la réception (2411) d'une position de référence de l'appareil de support de patient ;
la détermination (2413) d'un signal de réglage de position pour commander au moins un actionneur afin de régler la position de l'appareil de positionnement de patient d'une position actuelle à la position de référence sur la base d'un paramètre dépendant d'une caractéristique physique d'un patient,
dans lequel le signal de réglage de position est en outre basé sur une divergence entre une position estimée et la position de référence,
dans lequel la position estimée est estimée au moins partiellement sur la base d'un relevé en provenance d'un capteur,
dans lequel le paramètre dépendant d'une caractéristique physique du patient est déterminé à l'aide de signaux reçus en provenance du capteur.

2. Procédé (2410) selon la revendication 1, dans lequel la caractéristique physique du patient comprend au moins une caractéristique parmi un poids de patient et une taille de patient.

3. Procédé (2410) selon la revendication 1 ou la revendication 2, dans lequel la divergence est provoquée par un mouvement de rotation de l'appareil de support de patient inclinable,
facultativement dans lequel signal de réglage de position est agencé pour compenser la divergence en provoquant un mouvement de translation de l'appareil de support de patient inclinable.

4. Procédé (2410) selon l'une quelconque des revendications 1 à 3, dans lequel le capteur est un inclinomètre monté sur l'appareil de support de patient inclinable.

5. Procédé (2410) selon l'une quelconque des revendications précédentes, dans lequel le paramètre dépendant d'une caractéristique physique du patient est déterminé à partir de la différence entre un premier relevé de capteur et un second relevé de capteur, le premier relevé de capteur étant pris au niveau d'une première position de l'appareil de support de patient inclinable, le second relevé de capteur étant pris au niveau d'une seconde position de l'appareil de support de patient inclinable, dans lequel le patient est positionné sur l'appareil de support de patient inclinable pendant chaque relevé de capteur.

6. Procédé (2410) selon l'une quelconque des revendications 1 à 5, dans lequel la divergence entre la position estimée et la position de référence est provoquée par déformation de l'appareil de support de patient,
facultativement dans lequel la déformation de l'appareil de support de patient se produit lorsqu'un patient est présent sur l'appareil de support de patient.

7. Procédé (2410) selon la revendication 6, dans lequel la déformation de l'appareil de support de patient provoque une divergence entre la position estimée et la position de référence dans une direction verticale.

8. Procédé (2410) selon l'une quelconque des revendications précédentes, dans lequel le signal de réglage de position est agencé pour provoquer un mouvement de rotation de tangage de l'appareil de support de patient inclinable.

9. Procédé (2410) selon l'une quelconque des revendications précédentes, le procédé comprenant en outre la commande de l'au moins un actionneur à l'aide du signal de réglage de position.

10. Procédé (2410) selon l'une quelconque des revendications précédentes, dans lequel le procédé comprend la détermination d'un signal de réglage de position de telle sorte qu'une précision de positionnement isocentrique dans l'ensemble de l'appareil de positionnement de patient corresponde à une sphère avec un rayon inférieur à 0,5 mm.

11. Procédé (2410) selon l'une quelconque des revendications précédentes, dans lequel au moins :
A. le signal de réglage de position est déterminé à partir du paramètre dépendant de la caractéristique physique du patient à l'aide d'une table de consultation ; et/ou
B. le signal de réglage de position est agencé pour compenser le poids d'un patient.

12. Dispositif de radiothérapie comprenant un processeur configuré pour réaliser le procédé (2410) selon l'une quelconque des revendications précédentes.

13. Support lisible par ordinateur contenant des instructions qui, lorsqu'elles sont exécutées par un processeur, amènent un système de radiothérapie à réaliser le procédé (2410) selon l'une quelconque des revendications 1 à 11.
